# EUROPEAN PATENT SPECIFICATION

(11) **EP 1 855 759 B1**
(45) Date of publication and mention of the grant of the patent: **15.03.2017**
(21) Application number: 05810308.6
(22) Date of filing: 06.10.2005
(51) Int. Cl.: A61N 7/02, A61B 8/08

(54) **SYSTEM FOR ULTRASOUND TISSUE TREATMENT**
SYSTEM FÜR DIE ULTRASCHALLBEHANDLUNG VON GEWEBE
SYSTEME DE TRAITEMENT DE TISSUS PAR ULTRASONS

(30) Priority: 06.10.2004 US 616752 P; 06.10.2004 US 616755 P; 07.10.2004 US 617203 P; 07.10.2004 US 617295 P
(43) Date of publication of application: 21.11.2007
(60) Divisional application: 11172024.9
(73) Proprietor: Guided Therapy Systems, L.L.C., Mesa, Arizona 85202-1150 (US)
(72) Inventor: BARTHE, Peter G., Phoenix, Arizona 85048 (US); SLAYTON, Michael H., Tempe, Arizona 85253 (US); MAKIN, Inder Raj S., Mesa, Arizona 85215 (US); O'CONNOR, Brian D., Phoenix, Arizona 85021 (US)
(74) Representative: Ricker, Mathias
(86) International application number: PCT/US2005/036377
(87) International publication number: WO 2006/042201

(56) References cited:
- DE-A1- 10 219 297
- GB-A- 2 113 099
- US-A- 4 979 501
- US-A- 5 143 074
- US-A- 5 520 188
- US-A- 5 558 092
- US-B1- 6 623 430

## Description

### Field of Invention

The present invention relates to ultrasound therapy and imaging systems, and in particular to a system for noninvasive tissue treatment, such as for use in face lifts and deep tissue tightening, and/or in treatment of photoaged tissue, acne and sebaceous glands, and sweat glands.

### Background of the Invention

Coarse sagging of the skin and facial musculature occurs gradually over time due to gravity and chronic changes in connective tissue generally associated with aging. Invasive surgical treatment to tighten such tissues is common, for example by facelift procedures. In these treatments for connective tissue sagging, a portion of the tissue is usually removed, and sutures or other fasteners are used to suspend the sagging tissue structures. On the face, the Superficial Muscular Aponeurosis System (SMAS) forms a continuous layer superficial to the muscles of facial expression and beneath the skin and subcutaneous fat. Conventional face lift operations involve suspension of the SMAS through such suture and fastener procedures.

No present procedures have been developed yet, which provide the combination of targeted, precise, local heating to a specified temperature region capable of inducing ablation (thermal injury) to underlying skin and subcutaneous fat. Attempts have included the use of radio frequency (RF) devices that have been used to produce heating and shrinkage of skin on the face with some limited success as a non-invasive alternative to surgical lifting procedures. However, RF is a dispersive form of energy deposition. RF energy is impossible to control precisely within the heated tissue volume and depth, because resistive heating of tissues by RF energy occurs along the entire path of electrical conduction through tissues. Another restriction of RF energy for non-invasive tightening of the SMAS is unwanted destruction of the overlying fat and skin layers. The electric impedance to RF within fat, overlying the suspensory connective structures intended for shrinking, leads to higher temperatures in the fat than in the target suspensory structures. Similarly, mid-infrared lasers and other light sources have been used to non-invasively heat and shrink connective tissues of the dermis, again with limited success. However, light is not capable of non-invasive treatment of SMAS because light does not penetrate deeply enough to produce local heating there. Below a depth of approximately 1 mm, light energy is multiply scattered and cannot be focused to achieve precise local heating.

In addition to sagging of skin and facial musculature regions being a concern to aging individuals, photoaging of human skin is a complex response due to inflammation, oxidative injury, cellular and extracellular changes induced by decades of sunlight exposure. UV wavelengths are thought to be mainly responsible. Both of the primary skin layers, epidermis and dermis, are affected. Epidermal photoaging includes pigmentary lesions called ephilides (freckles) and solar lentigines (larger pigmented spots), plus pre-cancerous clonal lesions of keratinocytes called actinic keratoses. Thermal destruction of part or all of the epidermis, the outermost cellular layer of skin about 0.1 mm thick, is an effective treatment for epidermal photoaging. For example, lasers that vaporize epidermis are highly effective in a treatment called laser resurfacing. However laser resurfacing creates a significant skin wound with risk of infection, and prolonged healing. Dermal changes of photoaging include solar elastosis (an accumulation of abnormally-formed elastin fibers in the upper reticular layer of the dermis), laxity, loss of elasticity, fine and coarse wrinkles. Laser resurfacing to a depth below the dermo-epidermal junction can be highly effective for improving dermal photoaging, through a process of stimulated wound healing. Deep chemical peels, dermabrasion and other methods of destruction of epidermis and/or dermis are also effective, and also produce a significant open skin wound with risk of infection and delayed healing.

Patterns of stimulated thermal damage to epidermis and/or dermis are also effective for treatment of photoaging. Recently, "fractional photothermolysis" using mid-infrared lasers to produce a microscopic array of thermal injury zones that include both epidermis and dermis was reported to be effective and well-tolerated for treatment of photoaging (D. Manstein et al. "Fractional Photothermolysis: a new concept for cutaneous remodeling using microscopic patterns of thermal injury." Lasers Surg Med 34:426-438, 2004). A primary advantage of fractional photothermolysis is that each zone of thermal injury is smaller than can be easily seen with the unaided eye, and surrounded by a zone of healthy tissue that initiates a rapid healing response. As described Manstein, the epidermis is stimulated to heal rapidly and without creating an open wound. The microscopic zones of thermally injured epidermis slough harmlessly from the skin surface after several days to several weeks, leaving a rejuvenated epidermis with less photoaging changes. Repeat treatments, which are well tolerated, can be performed until a desired result is obtained. The microscopic zones of thermal injury with fractional photothermolysis extend well into the dermis, as well. Dermis does not heal as rapidly as epidermis, in general. Over weeks to months following treatment, some of the abnormal dermis due to photoaging is remodeled, however, leading to improvement in laxity, wrinkles and skin texture.

Fractional photothermolysis (FP) is intrinsically limited to regions of approximately the upper 1-millimeter of skin. The basic concept of producing well-controlled arrays of thermal injury is therefore limited with fractional photothermolysis, to superficial aspects of photoaging. Aging, which also causes laxity of the skin, and photoaging involve deeper layers of the dermis. Solar elastosis can extend throughout the dermis, to approximately 3 mm deep or more. Laxity and loss of elasticity due to aging are bulk problems of the dermis.

A fundamental requirement for producing arrays of small thermal injury zones using a source of radiant energy that propagates and is absorbed within tissue, is that the source of radiant energy be capable of being adequately delivered to the tissue depth for which the array is desired. Near the skin surface, light can be used, as in fractional photothermolysis. However, light that propagates more than about 1 mm through skin has been multiplied scattered, and can no longer be focused or delivered.

Acne vulgaris is the most common skin disorder. Acne causes temporary and permanent disfigurement. Acne typically appears on the face, back and/or chest at the onset of adrenarchy, i.e. when sex hormone activity increases in both boys and girls near puberty. Acne is a disorder of hair follicles, in which a plug forms within the outflow tract of the hair follicle. Sebum, an oily product of sebaceous glands attached to each hair follicle, and cellular debris builds in the plug. Inflammation and often rupture of the hair follicles ensues, leading to gross inflammation, pus (a "whitehead"), pain, bleeding, and/or eventually scarring. If the acne lesion consists of an accumulated unruptured plug within the hair follicle, a "blackhead" forms. If the follicle ruptures superficially, a small pustule forms that often heals after a few weeks without scarring. If the follicle ruptures within the mid or deep dermis, a painful cystic abscess forms. Cystic acne usually heals with permanent and disfiguring scars.

The exact pathophysiology of acne is complex and is not fully understood. However, several basic elements are necessary to produce an acne lesion, and acne therapies are based on attacking one or more of these basic elements. First, an active sebaceous gland is necessary. The most potent treatments for acne are oral retinoids such as retinoic acid (Accutane), which inhibit sebaceous gland function. Sebaceous gland activity is driven by androgen and other sex steroid hormones. Women often experience cycle-dependent acne that may respond to treatment with birth control pills containing low amounts of progestins. Second, a plug must form in the outflow tract of the follicle, called the infundibulum. Bacteria, particularly *Proprionobacteria acnes (P acnes)* that digest sebum and follicular debris, contribute to plugging. Topical retinoids, mild acids and benzoyl peroxide are used as treatments to decrease follicular plugging. Antibiotics effective against P acnes are given either topically or orally; the prevalence of antibiotic-resistant P acnes is increasing. Third, inflammation is part of the process that breaks down the wall of a follicle containing plugs, leading to rupture of the follicle with release of irritating materials into the skin, abscess formation, and scarring. Anti-inflammatory agents including some antibiotics are helpful in treating acne.

The most potent treatment for acne at present is oral retinoid therapy. Unfortunately, this is a toxic and teratogenic treatment. Unplanned pregnancies in women taking Accutane lead to a high rate of fetal malformations. An aggressive program to prevent this in the US was implemented, but has failed to prevent the problem. Systemic retinoid treatment also causes major side effects including extreme dryness during treatment, risk of hepatitis, bone changes, mood changes, and others. The high effectiveness and high toxicity of oral retinoids for treatment of cystic acne strongly suggests that an alternative treatment that targets sebaceous glands is needed.

The sweat glands in the body are of divided into apocrine and eccrine glands. Apocrine glands are similar to sebaceous glands, and are present mainly in the axillae. These glands, like sebaceous glands, secrete an oily proteinaceous product into the follicles. Bacterial digestion of apocrine sweat is largely responsible for underarm "body odor". Similarly, eccrine sweat glands are present deep in the dermis in the palms, soles and armpits and are responsible for temperature regulation resulting from sweating. Excessive activity of these glands also results in copious amounts of abnormal sweating ("hyperhidrosis"), primarily under autonomic neuronal control. Reduction of sweating from under the armpits and other regions is a particularly desirable effect within the modern society. Presently, chemical antiperspirants and deodorants are used frequently as a matter of personal hygiene. Antiperspirants are aluminum based salts that block the sweat gland ducts. The deodorant changes the pH of the skin *milieu* thereby minimizing the presence of (smell inducing) bacteria. The effects with both these components however, are temporary and these chemicals are known to irritate the skin in a good percentage of users.

Further, there is currently a significant unmet need in managing the excessive sweating and concomitant issues with odor as a result of Hydradenitis suppurativa (irritable infected armpit). This acne-like process in apocine follicles also causes hydradenitis suppurativa, which is often a devastating condition in which very painful cysts and scarring occurs repeatedly in the axillae. The etiology (causes) of this clinical condition is not well understood. However, there are a number of marginally effective approaches to manage this condition. Retinoid drug therapy works marginally but is associated with severe toxicity. Some prescription formulations of antiperspirants can be used, but they are not particularly effective. These preparations can be applied with the addition of an iontophoretic device. This technique however, is not known to be any more effective than the formulation. The sweat glands can be surgically removed from the armpits and/or the sympathetic nerve supply can be interrupted surgically. This approach is fraught with its own drawbacks in terms of morbidity, scarring and cost. BOTOX® is being used ever more for paralyzing the nerve connections that induce excessive sweating in the armpits. However, this is a new approach yet to be completely validated. This technique requires multiple injections (painful) and the results last a few months only (3-4 months), hence need to be repeated. This technique does not get rid of the odor associated with the condition.
US Patent No. 5,558,092 discloses methods and apparatus for performing diagnostic ultrasound simultaneously with the application of therapeutic ultrasonic waves are disclosed. The methods and apparatus are particularly advantageous in performing ultrasonic imaging of a region of a patient while simultaneously applying therapeutic ultrasonic waves to the region for the purpose of rupturing vesicles administered to that region for purposes such as enhanced cavitation or the targeted release of a bioactive agent into the region. An operator is able to monitor the rupture of vesicles in real time.
US Patent No. 5,520,188 discloses a transducer for use in a localization and therapeutic ultrasound system. The transducer includes multiple elements that are driven separately. The elements operate together to focus a continuous wave ultrasound beam at a focal zone that is at a variable distance from the elements. The transducer includes a mechanism to adjust the focal distance so that the focal zone may be moved to multiple depths.
US Patent No. 6,623,430 discloses a method and apparatus for controlling the safe delivery of thermosensitive liposomes containing medicant to a targeted tissue region using ultrasound. Thermosensitive liposomes containing medicants are delivered to a region of interest, the region of interest is located using ultrasound imaging, ultrasound therapy is applied to heat the region of interest, and the temperature of the region is monitored to determine whether a designated threshold temperature has been reached which allows for the release of medicants from the liposomes. If the threshold temperature is reached, and the liposomes are melted, the treatment stops. If the threshold temperature has not been reached, the application of ultrasound therapy and ultrasound imaging are alternated until the threshold temperature is reached. The ultrasound imaging, temperature monitoring and ultrasound therapy are preferably performed with a single transducer.
GB 2,113,099 discloses that a selected portion of tissue to be treated is imaged using a pulse-echo ultrasound imaging system. This system comprises an ultrasound transducer which is driven during imaging at physiologically tolerable power levels. Once the imaging system is positioned to image the tissue portion to be treated, the power at which the transducer is driven is increased to a physiologically intolerable level which is sufficient to thermally treat that tissue portion. Following scarring, the tissue portion is again imaged to determine the effectiveness of the treatment. The preferred embodiment apparatus comprises a transducer-lens system for focusing ultrasound onto a preselected focal area, an imaging means for displaying images constructed from the echoes of said ultrasound, and a pulser means selectively operable at physiologically tolerable or intolerable power levels to selectively image or to cause thermal treatment of tissue in the focal area.
US Patent No. 5,143,074 discloses an ultrasonic treatment device comprising a power transducer in the form of a spherical cup serving both as treatment wave generator and as echographic transceiver, wherein the transducer is caused to oscillate (motor 2) during the treatment, so as to obtain sectorial B type scanning and it is excited (circuits 1 to 14) with treatment waves only in a restricted angular scanning sector and with echographic waves in the rest of the scanned sector.
DE 102 19297 A1 discloses a method and device for generation of scar tissue in biological soft tissue by use of electromagnetic energy, whereby said scar tissue is at least two-dimensional. The device used combines a laser, for generation of the electromagnetic energy, with an ultrasonic transducer for application of ultrasonic energy with backscatter ultrasound used for an A-scan evaluation. Accordingly the laser optical waveguide also forms a transfer path for the sound energy.
US Patent No. 4,979,501 discloses a method for medically treating a patient suffering from a pathological bone condition of a limb, which comprises the steps of: anesthetizing the patient; fixing the limb affected with the pathological bone condition and centering its pathological site; treating the pathological site, once, consecutively, and extracorporeally with impact waves of from 300 to 600 impacts with a frequency of impacts of from 0.5 to 4 per second at a pressure of from 700 to 2500 bars and a pulse duration of from 0.5 to 4 microseconds for a period of 10 to 120 minutes; and subsequently immobilizing the limb for a period of from 15 to 90 days.

### Summary of the Invention

Accordingly, the present application provides an ultrasound treatment system in accordance with independent claim 1. Advantageous features, variations, and embodiments are provided in dependent claims.

A system for noninvasive tissue treatment, such as for use in face lifts and deep tissue tightening is provided. For example, for facilitating face lifts and deep tissue tightening, an exemplary treatment system can be configured for the imaging, monitoring, and thermal injury to treat the SMAS region. In accordance with an exemplary embodiment, the exemplary system is configured for treating the SMAS region by first, imaging of the region of interest for localization of the treatment area and surrounding structures, second, delivery of ultrasound energy at a depth, distribution, timing, and energy level to achieve the desired therapeutic effect, and third to monitor the treatment area before, during, and after therapy to plan and assess the results and/or provide feedback.

In accordance with an exemplary embodiment, an exemplary treatment system comprises an imaging/therapy probe, a control system and display system. The imaging/therapy probe can comprise various probe and/or transducer configurations. For example, the probe can be configured for a combined dual-mode imaging/therapy transducer, coupled or co-housed imaging/therapy transducers, or simply a therapy probe and an imaging probe. The control system and display system can also comprise various configurations for controlling probe and system functionality, including for example a microprocessor with software and a plurality of input/output devices, a system for controlling electronic and/or mechanical scanning and/or multiplexing of transducers, a system for power delivery, systems for monitoring, systems for sensing the spatial position of the probe and/or transducers, and systems for handling user input and recording treatment results, among others.

In accordance with an exemplary embodiment, ultrasound imaging can be utilized for safety purposes, such as to avoid injuring vital structures such as the facial nerve (motor nerve), parotid gland, facial artery, and trigeminal nerve (for sensory functions) among others. For example, ultrasound imaging can be used to identify SMAS as the superficial layer well defined by echoes overlying the facial muscles. Such muscles can be readily seen and better identified by moving them, and their image may be further enhanced via signal and image processing. In accordance with an exemplary embodiment, ultrasound therapy via focused ultrasound from a single element is utilized to treat the SMAS region at fixed and/or variable depth or dynamically controllable depths and positions.

### Brief Description of the Drawings

The subject matter of the invention is particularly pointed out in the concluding portion of the specification. The invention, however, both as to organization and method of operation, may best be understood by reference to the following description taken in conjunction with the accompanying drawing figures, in which like parts may be referred to by like numerals:
FIG. 1 illustrates a block diagram of a treatment system in accordance with an exemplary embodiment of the present invention;
FIGS. 2A-2Q illustrates schematic diagrams of an ultrasound imaging/therapy and monitoring system for treating tissue in accordance with various exemplary embodiments of the present invention;
FIGS. 3A and 3B illustrate block diagrams of an exemplary control system in accordance with exemplary embodiments of the present invention;
FIGS. 4A and 4B illustrate block diagrams of an exemplary probe system in accordance with exemplary embodiments of the present invention;
FIG. 5 illustrates a cross-sectional diagram of an exemplary transducer, not forming part of the present invention;
FIGS. 6A and 6B illustrate cross-sectional diagrams of an exemplary transducer, not forming part of the present invention;
FIG. 7 illustrates exemplary transducer configurations for ultrasound treatment, not all forming part of the present invention;
FIGS. 8A and 8B illustrate cross-sectional diagrams of an exemplary transducer in accordance with another exemplary embodiment of the present invention;
FIG. 9 illustrates an exemplary transducer configured as a two-dimensional array for ultrasound treatment, not forming part of the present invention;
FIGS. 10A-10F illustrate cross-sectional diagrams of exemplary transducers, not forming part of the present invention;
FIG. 11 illustrates a schematic diagram of an acoustic coupling and cooling system in accordance with an exemplary embodiment of the present invention;
FIG. 12 illustrates a block diagram of a treatment system comprising an ultrasound treatment subsystem combined with additional subsystems of treatment monitoring and/or treatment imaging as well as a secondary treatment subsystem in accordance with an exemplary embodiment of the present invention; and
FIG. 13 illustrates a schematic diagram with imaging, therapy, or monitoring being provided with one or more active or passive oral inserts in accordance with an exemplary embodiment of the present invention.

### Detailed Description

The present invention may be described herein in terms of various functional components and processing steps. It should be appreciated that such components and steps may be realized by any number of hardware components configured to perform the specified functions. For example, the present invention may employ various medical treatment devices, visual imaging and display devices, input terminals and the like, which may carry out a variety of functions under the control of one or more control systems or other control devices. In addition, the present invention may be practiced in any number of medical contexts and the exemplary embodiments relating to a method and system for noninvasive face lift and deep tissue tightening, photoaged tissue, acne and sebaceous glands, and sweat glands. as described herein are merely indicative of exemplary applications for the invention. For example, the principles, features and methods discussed may be applied to any muscular fascia, gland or other tissue region or any other medical application. Further, various aspects of the present invention may be suitably applied to other applications.

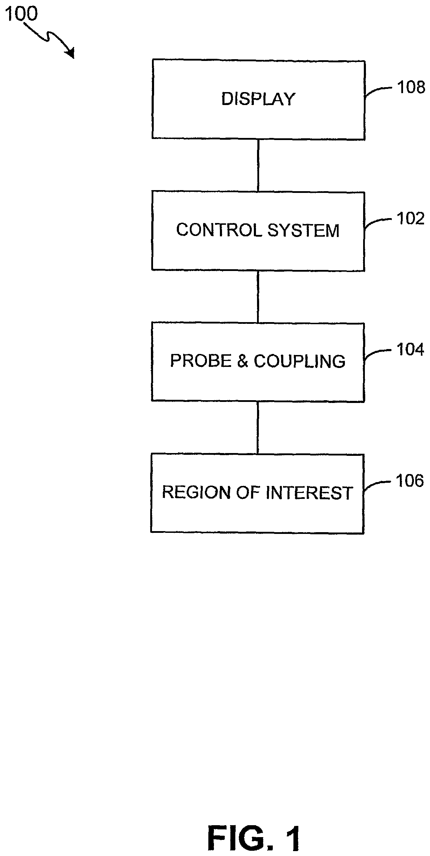

In accordance with various aspects of the present invention, a system for tissue treatment is provided. For example, in accordance with an exemplary embodiment, with reference to Figure 1, an exemplary treatment system 100 configured to treat a region of interest 106 comprises a control system 102, an imaging/therapy probe with acoustic coupling 104, and a display system 108. Control system 102 and display system 108 can comprise various configurations for controlling probe 102 and overall system 100 functionality, such as, for example, a microprocessor with software and a plurality of input/output devices, system and devices for controlling electronic and/or mechanical scanning and/or multiplexing of transducers, a system for power delivery, systems for monitoring, systems for sensing the spatial position of the probe and/or transducers, and/or systems for handling user input and recording treatment results, among others. Imaging/therapy probe 104 can comprise various probe and/or transducer configurations. For example, probe 104 can be configured for a combined dual-mode imaging/therapy transducer, coupled or co-housed imaging/therapy transducers, or simply a separate therapy probe and an imaging probe.

In accordance with an exemplary embodiment, treatment system 100 is configured for treating the tissue region by first, imaging of region of interest 106 for localization of the treatment area and surrounding structures, second, delivery of ultrasound energy at a depth, distribution, timing, and energy level to achieve the desired therapeutic effect, and third to monitor the treatment area before, during, and after therapy to plan and assess the results and/or provide feedback.

As to the treatment of face lifts, the SMAS region and connective tissue can be permanently tightened by thermal treatment to temperatures about 60 degrees C or higher. Upon ablating, collagen fibers shrink immediately by approximately 30% of their length. The shrunken fibers can produce tightening of the tissue, wherein the shrinkage should occur along the dominant direction of the collagen fibers. Throughout the body, collagen fibers are laid down in connective tissues along the lines of chronic stress (tension). On the aged face, the collagen fibers of the SMAS region are predominantly oriented along the lines of gravitational tension. Shrinkage of these fibers results in tightening of the SMAS in the direction desired for correction of laxity and sagging due to aging. The treatment comprises the ablation of specific regions of the SMAS region and similar suspensory connective tissues.

In addition, the SMAS region varies in depth and thickness at different locations, e.g., between 0.5 mm to 5mm or more. On the face, important structures such as nerves, parotid gland, arteries and veins are present over, under or near the SMAS region. Tightening of the SMAS in certain locations, such as the preauricular region associated with sagging of the cheek to create jowls, the frontal region to associated with sagging brows, mandibular region associated with sagging neck, can be conducted. Treating through localized heating of regions of the SMAS or other suspensory subcutaneous connective tissue structures to temperatures of about 60-90 °C, without significant damage to overlying or distal/underlying tissue, i.e., proximal tissue, as well as the precise delivery of therapeutic energy to SMAS regions, and obtaining feedback from the region of interest before, during, and after treatment can be suitably accomplished through treatment system 100.

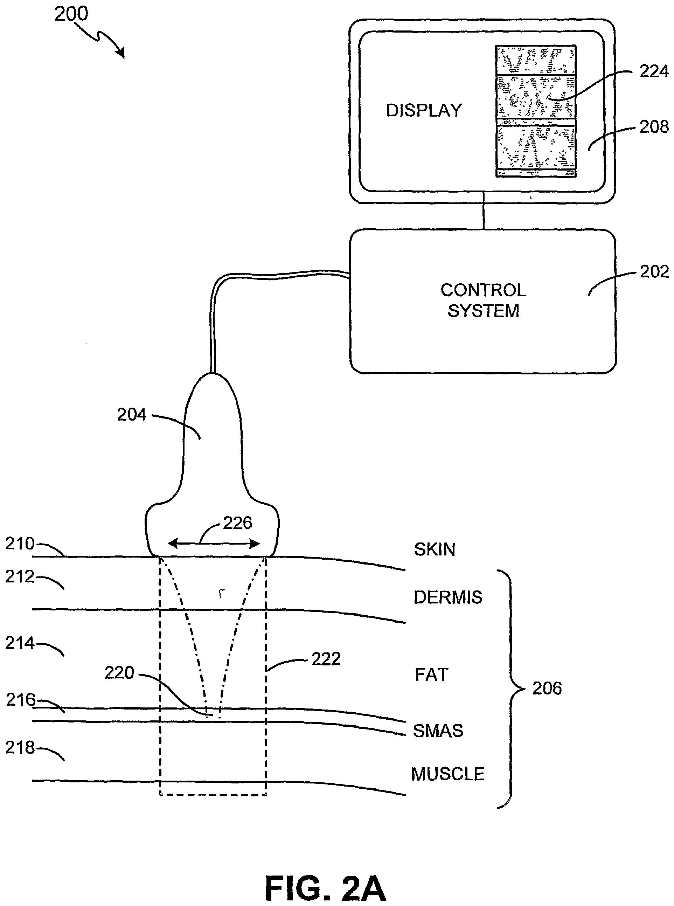

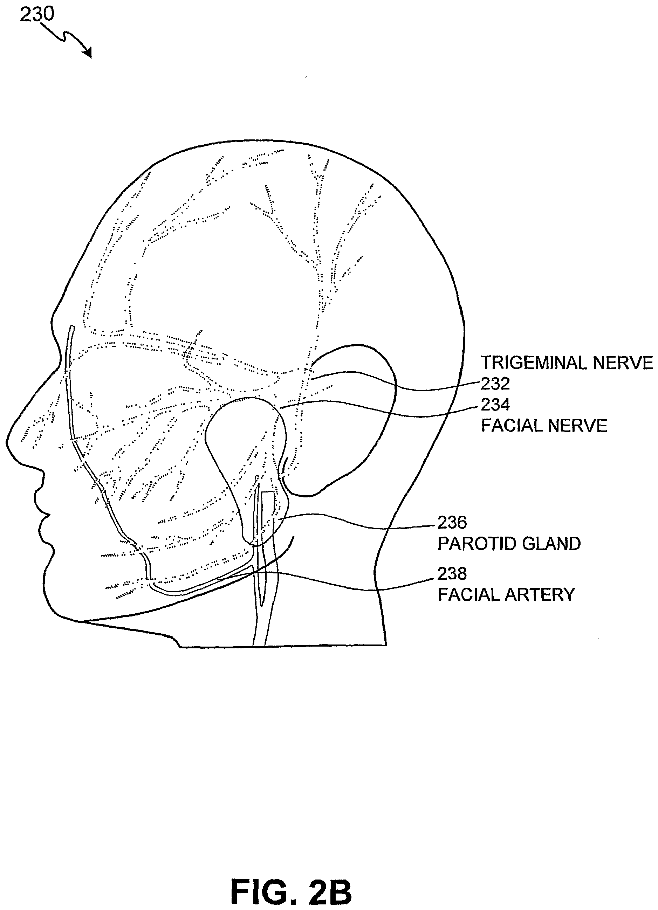

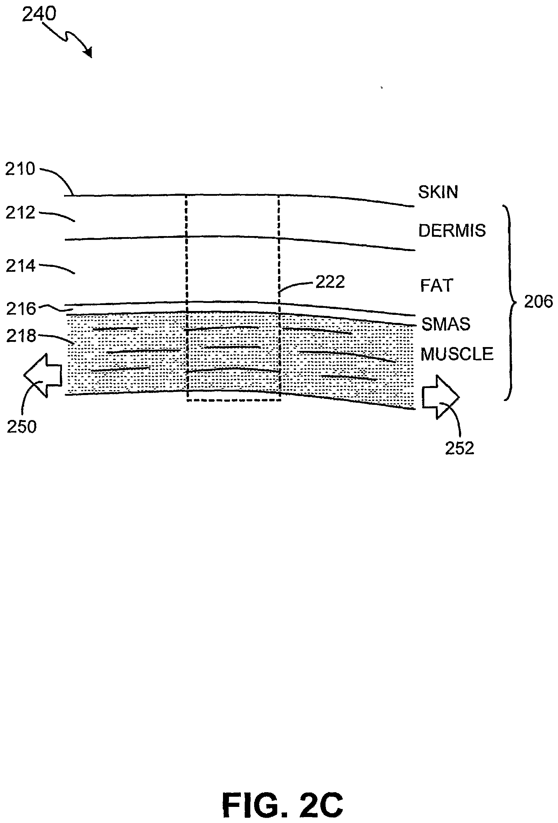

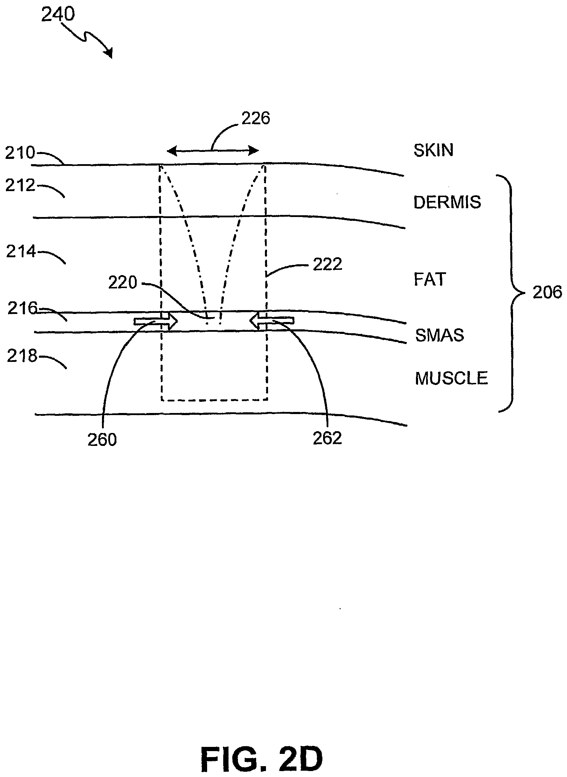

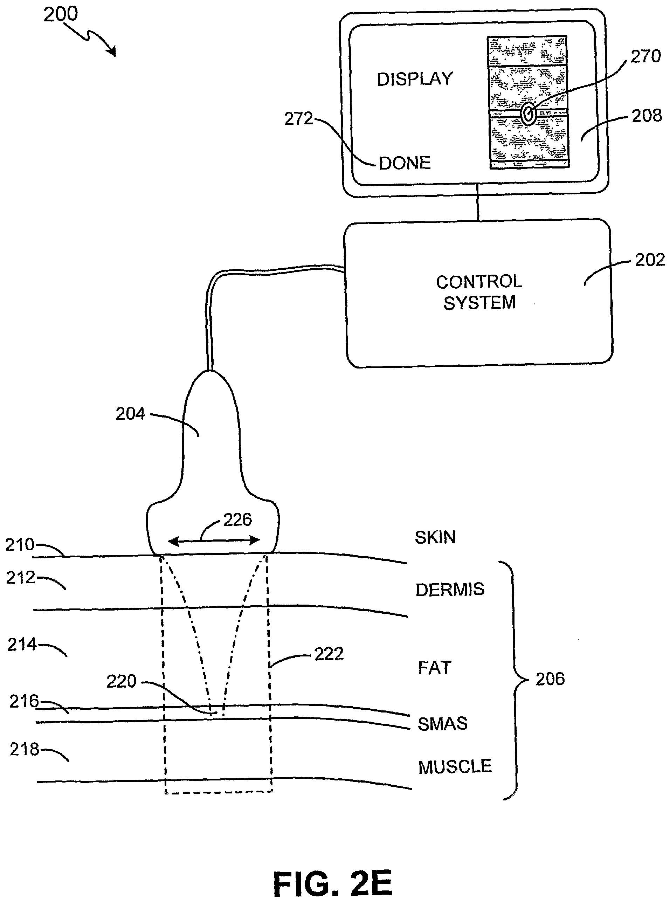

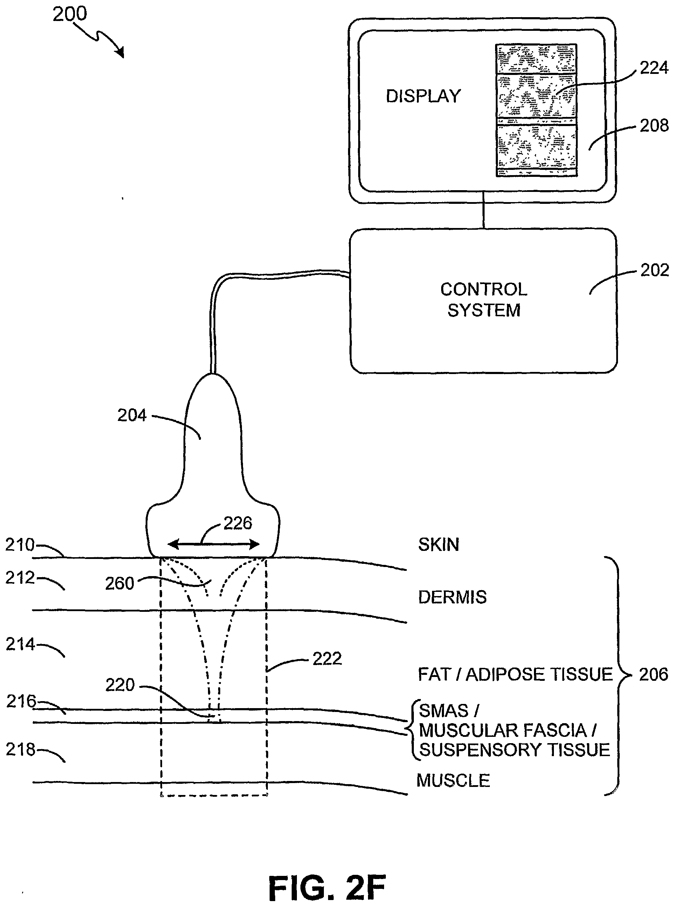

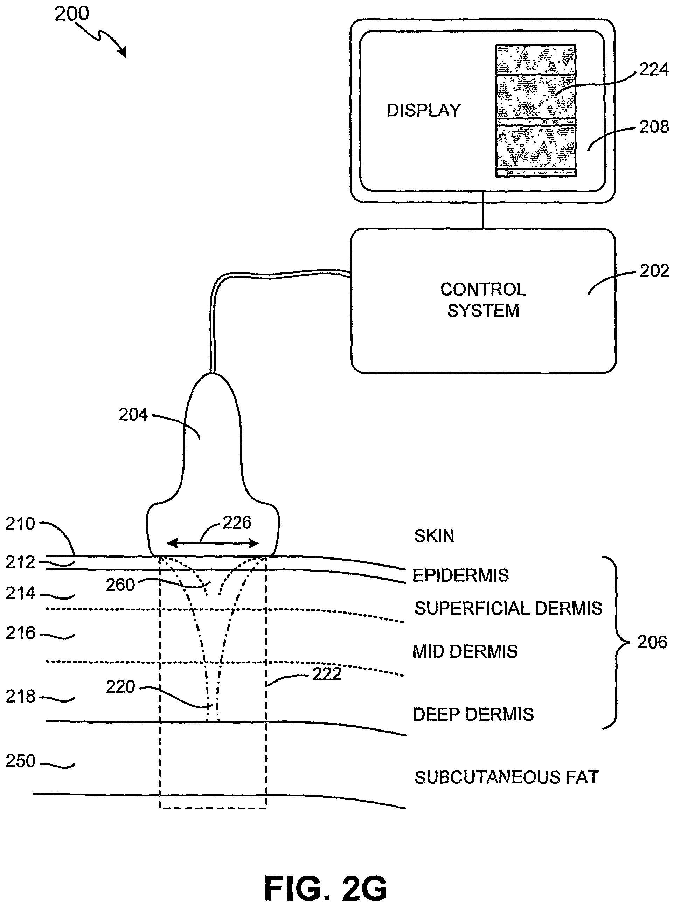

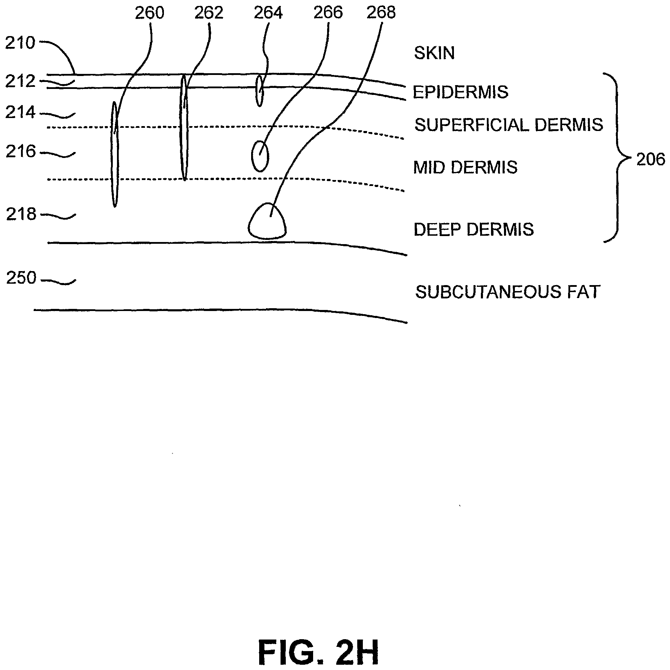

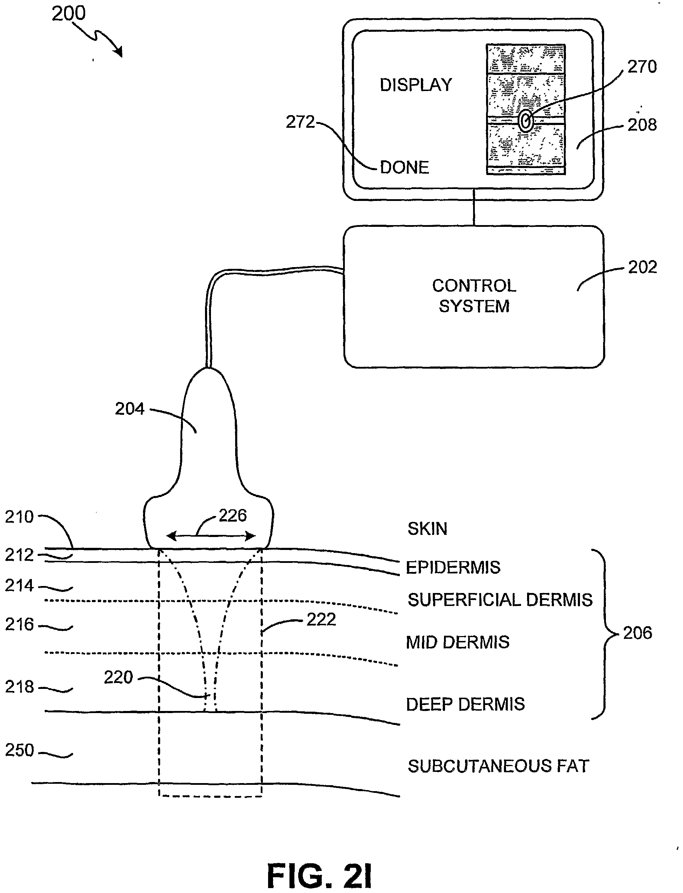

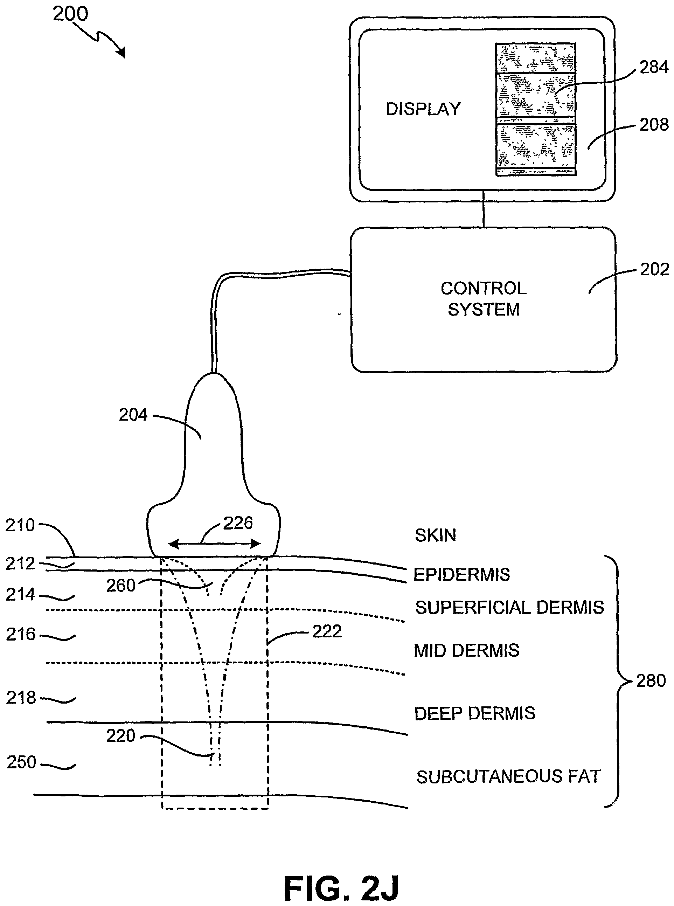

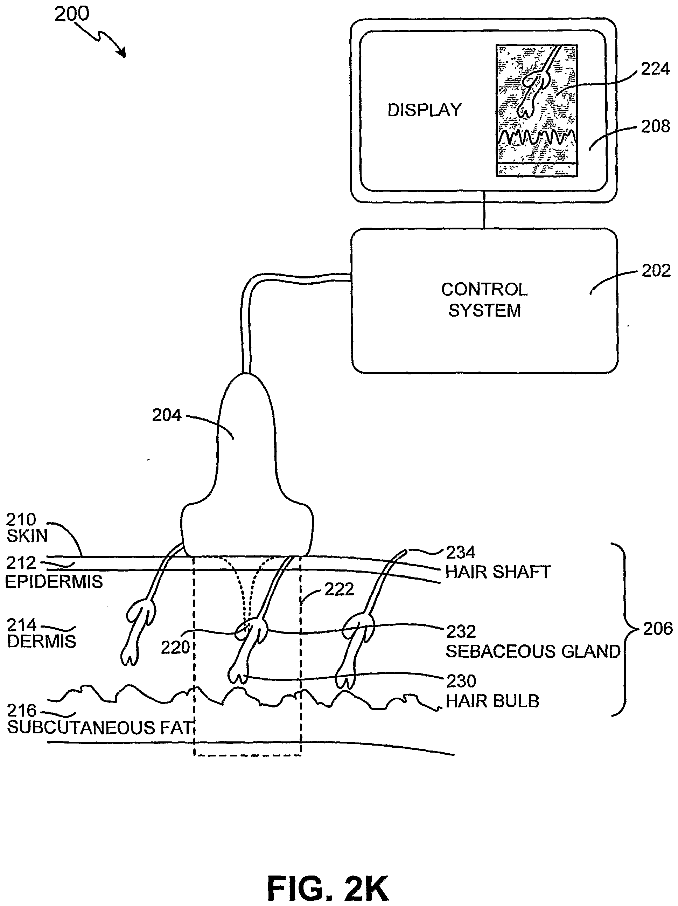

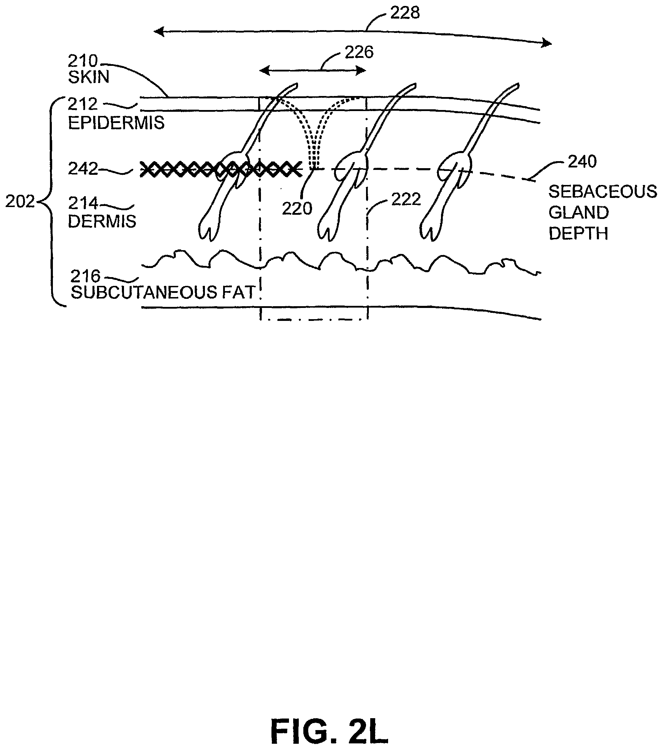

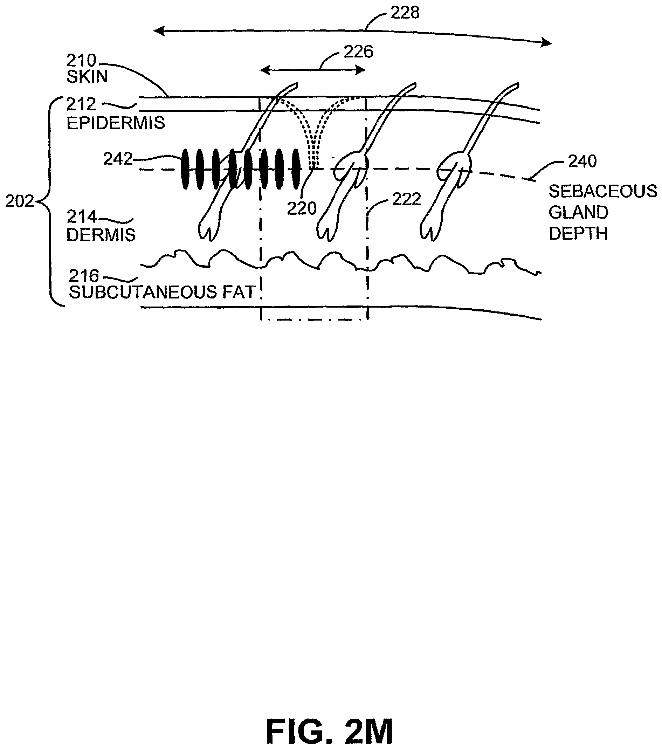

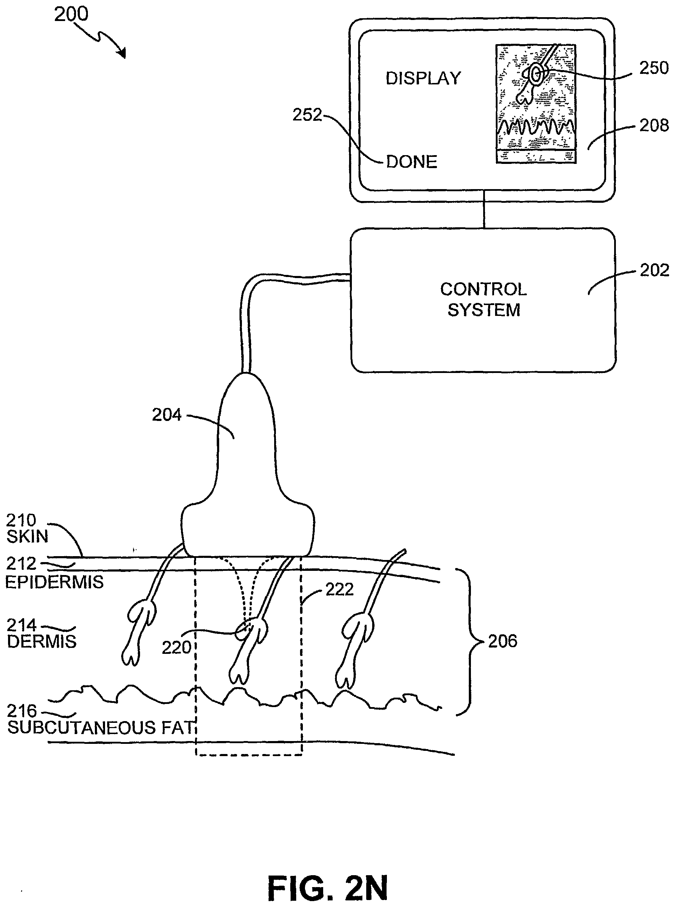

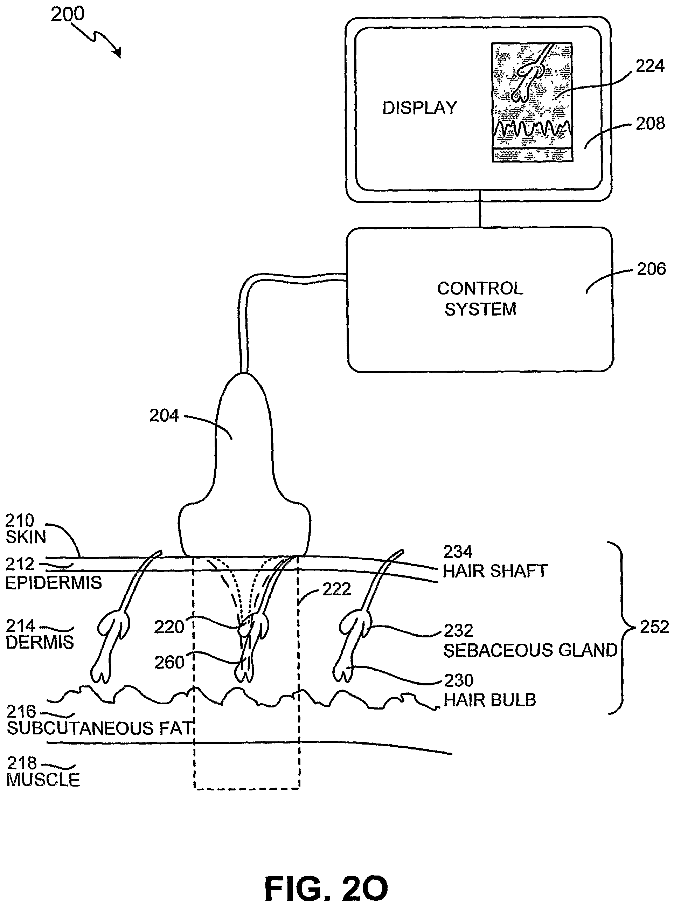

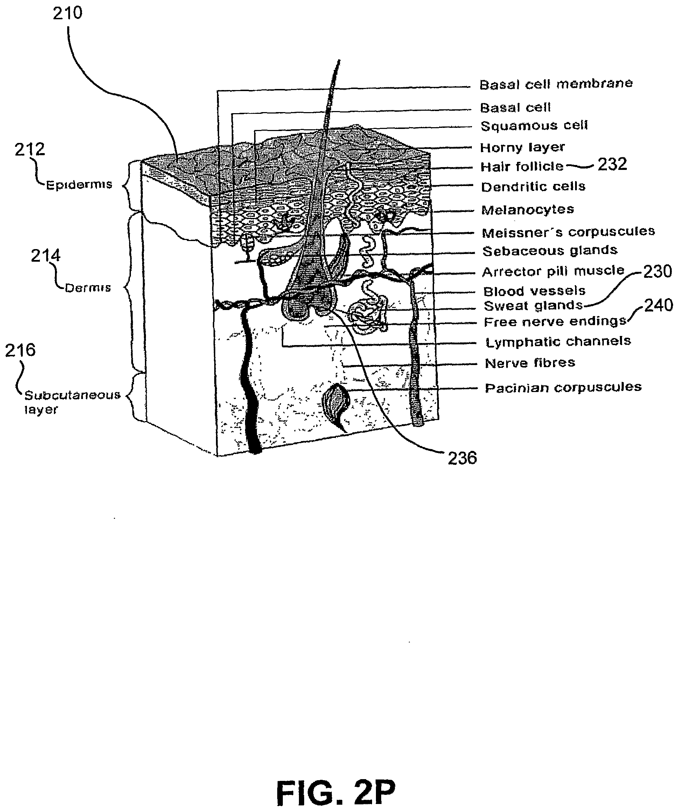

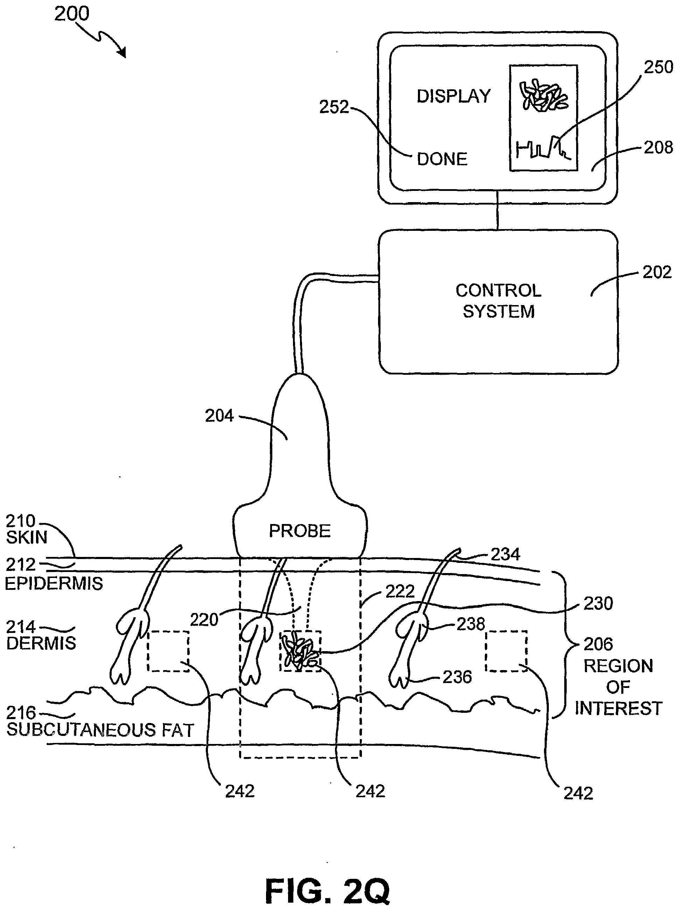

To further illustrate an exemplary method and system 200, with reference to Figure 2, imaging of a region of interest 206, such as by imaging a region 222 and displaying images 224 of the region of interest 206 on a display 208, to facilitate localization of the treatment area and surrounding structures can initially be conducted. Next, delivery of ultrasound energy 220 at a suitably depth, distribution, timing, and energy level to achieve the desired therapeutic effect of thermal injury or ablation to treat SMAS region 216 can be suitably provided by probe 204 through control by control system 202. Monitoring of the treatment area and surrounding structures before, during, and after therapy, i.e., before, during, and after the delivery of ultrasound energy to SMAS region 216, can be provided to plan and assess the results and/or provide feedback to control system 202 and a system user.

Ultrasound imaging and providing of images 224 can facilitate safe targeting of the SMAS layer 216. For example, with reference to Fig. 2B, specific targeting for the delivery of energy can be better facilitated to avoid heating vital structures such as the facial nerve (motor nerve) 234, parotid gland (which makes saliva) 236, facial artery 238, and trigeminal nerve (for sensory functions) 232 among other regions. Further, use of imaging with targeted energy delivery to provide a limited and controlled depth of treatment can minimize the chance of damaging deep structures, such as for example, the facial nerve that lies below the parotid, which is typically 10 mm thick.

In accordance with an exemplary embodiment, with reference to Figure 2C, ultrasound imaging of region 222 of the region of interest 206 can also be used to delineate SMAS layer 216 as the superficial, echo-dense layer overlying facial muscles 218. Such muscles can be seen via imaging region 222 by moving muscles 218, for example by extensional flexing of muscle layer 218 generally towards directions 250 and 252. Such imaging of region 222 may be further enhanced via signal and image processing. Once SMAS layer 216 is localized and/or identified, SMAS layer 216 is ready for treatment.

The delivery of ultrasound energy 220 at a suitably depth, distribution, timing, and energy level is provided by probe 204 through controlled operation by control system 202 to achieve the desired therapeutic effect of thermal injury to treat SMAS region 216. During operation, probe 204 can also be mechanically and/or electronically scanned within tissue surface region 226 to treat an extended area. In addition, spatial control of a treatment depth 220 can be suitably adjusted in various ranges, such as between a wide range of approximately 0 to 15 mm, suitably fixed to a few discrete depths, with an adjustment limited to a fine range, e.g. approximately between 3 mm to 9 mm, and/or dynamically adjusted during treatment, to treat SMAS layer 216 that typically lies at a depth between approximately 5 mm to 7 mm. Before, during, and after the delivery of ultrasound energy to SMAS region 216, monitoring of the treatment area and surrounding structures can be provided to plan and assess the results and/or provide feedback to control system 202 and a system user.

For example, in accordance with an exemplary embodiment, with additional reference to Figure 2D, ultrasound imaging of region 222 can be used to monitor treatment by watching the amount of shrinkage of SMAS layer 216 in direction of areas 260 and 262, such as in real time or quasi-real time, during and after energy delivery to region 220. The onset of substantially immediate shrinkage of SMAS layer 216 is detectable by ultrasound imaging of region 222 and may be further enhanced via image and signal processing. The monitoring of such shrinkage can be ideal because it can confirm the intended therapeutic goal of noninvasive lifting and tissue tightening; in addition, such monitoring may be used for system feedback. In addition to image monitoring, additional treatment parameters that can be suitably monitored in accordance with various other exemplary embodiments may include temperature, video, profilometry, strain imaging and/or gauges or any other suitable spatial, temporal and/or other tissue parameters.

For example, in accordance with an exemplary embodiment of the present invention, with additional reference to Figure 2E, an exemplary monitoring method and system 200 may suitably monitor the temperature profile or other tissue parameters of the region of interest 206, such as attenuation or speed of sound of treatment region 222 and suitably adjust the spatial and/or temporal characteristics and energy levels of ultrasound therapy transducer probe 204. The results of such monitoring techniques may be indicated on display 208 in various manners, such as, for example, by way of one-, two-, or three-dimensional images of monitoring results 270, or may comprise an indicator 272, such as a success, fail and/or completed/done type of indication, or combinations thereof.

In accordance with another exemplary embodiment, with reference to Figure 2F, the targeting of particular region 220 within SMAS layer 216 can be suitably be expanded within region of interest 206 to include a combination of tissues, such as skin 210, dermis 212, fat /adipose tissue 214, SMAS / muscular fascia / and/or other suspensory tissue 216, and muscle 218. Treatment of a combination of such tissues and/or fascia may be treated including at least one of SMAS layer 216 or other layers of muscular fascia in combination with at least one of muscle tissue, adipose tissue, SMAS and/or other muscular fascia, skin, and dermis, can be suitably achieved by treatment system 200. For example, treatment of SMAS layer 216 may be performed in combination with treatment of dermis 280 by suitable adjustment of the spatial and temporal parameters of probe 204 within treatment system 200.

As to the treatment of photoaged tissue, it is desirable to be able to produce well-controlled arrays of microscopic zones of thermal injury not only near the surface of skin, but in the mid-dermis, and/or in the deep dermis. Thermal ablation of dermis at temperatures greater than about 60 °C, capable of producing denaturation of tissue, is also desirable in such arrays of thermal lesions. Shrinkage of dermis due to thermal action results from tightening of the skin during laser resurfacing.

In contrast to optical or RF approaches, ultrasound energy propagates as a wave with relatively little scattering, over depths up to many centimeters in tissue depending on the ultrasound frequency. The focal spot size achievable with any propagating wave energy, depends on wavelength. Ultrasound wavelength is equal to the acoustic velocity divided by the ultrasound frequency. Attenuation (absorption, mainly) of ultrasound by tissue also depends on frequency.

In accordance with an exemplary embodiment, the use of focused, unfocused, or defocused ultrasound for treatment of epidermal, superficial dermal, dermal, mid-dermal, and deep dermal components of photoaged tissue through adjustment of the strength, depth, and type of focusing, energy levels and timing cadence. For example, focused ultrasound can be used to create precise arrays of microscopic thermal ablation zones which have several advantages over fractional photothermolysis (FP). At high frequency and with superficial focusing or diffraction pattern, ultrasound ablation can mimic FP but utilize a simpler ablation device. Unlike fractional photothermolysis, ultrasound can produce an array of ablation zones much deeper into the skin or even into subcutaneous structures. Detection of changes in the reflection of ultrasound can be used for feedback control to detect a desired effect on the tissue and used to control the exposure intensity, time, and/or position.

To further illustrate the use of ultrasound for the treatment of photoaged tissue, with reference to Fig. 2G, an exemplary method and system are configured for initially imaging a region 222 of a region of interest 206 and displaying that region 224 during the localization of the treatment area and surrounding structures. After localization, delivery of ultrasound energy 220 at a depth, distribution, timing, and energy level to achieve the desired therapeutic effect of thermal ablation to treat an epidermis layer 212, superficial dermis layer 214, mid-dermis layer 216, and/or deep dermis layer 218 can be provided. Before, during, and after therapy, i.e., before, during, and after the delivery of ultrasound energy 220, exemplary method and system 200 can suitably monitor the treatment area and surrounding structures to plan and assess the results and/or provide feedback to control system 202 and/or a system user.

While an imaging function may be configured within control system 202 to facilitate imaging a region of interest, in accordance with another exemplary embodiment, an exemplary treatment system 200 may also be configured for therapy only or therapy and monitoring, without imaging functions. In such a case prior known depth of the region of interest, approximately 0 to 5 mm or less, is employed to achieve treatment zones in photoaged skin.

Probe 204 and/or transducers within can be mechanically and/or electronically scanned in a direction 226 to place treatment zones 260 over an extended area, such as a line to generate a matrix of closely spaced treatment spots. Treatment depth 220 can be adjusted between a range of approximately 0 to 5 mm, or otherwise until the depth of the deep dermis. Treatment may be confined to a fixed depth or a few discrete depths, or can be adjustment limited to a fine range, e.g. from approximately between 0 to 5 mm or the greatest depth of the deep dermis, or can be dynamically adjusted during treatment, to the treat region of interest 206 that lies above subcutaneous fat region 250.

In accordance with another exemplary embodiment of the present invention, with reference to Figure 2H, a treated zone 260 may extend throughout regions of the dermis, and may even extend to the epidermis, 262. In addition, as a treated zone increases in depth its cross section may increase from small size 264 (sub millimeter) in a shallow region near or at the epidermis, to medium size 266 (sub millimeter to millimeter sized) in a middle zone near or at the mid dermis, to large size 268 (millimeter sized) in deep zones near or at the deep dermis. Furthermore a single treated zone can have a shape expanding in cross section with depth, and/or be composed of the fusion of several smaller treatment zones. Spacing of treatment zones can be on the order of the treatment zone size. The ultrasound beam can be spatially and/or temporally controlled by changing the position of the transducer, its frequency, treatment depth, drive amplitude, and timing via the control system.

In accordance with another exemplary embodiment of the present invention, with reference to Fig. 2I, an exemplary treatment method and system 200 may be configured to monitor the temperature profile or other tissue parameters of region of interest 206, such as attenuation or speed of sound of the treatment region and suitably adjust the spatial and/or temporal characteristics and energy levels of the ultrasound therapy transducer. The results of such monitoring techniques may be indicated on display 208, such as through display of one-, two-, or three-dimensional images of monitoring results 270, or may comprise an indicator 272, such as a success, fail and/or completed/done type of indication, or combinations thereof. Additional treatment monitoring methods may be based on one or more of temperature, video, profilometry, strain imaging and/or gauges or any other suitable sensing method.

In accordance with another exemplary embodiment, with reference to Figure 2J, an expanded region of interest 280 can suitably include a combination of tissues, such as subcutaneous fat / adipose tissue 250. A combination of such tissues includes at least one of epidermis 212, superficial dermis 214, mid dermis 216, or deep dermis 218, in combination with at least one of muscle tissue, adipose tissue, or other tissues useful for treatment. For example, treatment 260 of superficial dermis may be performed in combination with treatment 220 of subcutaneous fat 250 by suitable adjustment of the spatial and temporal parameters of transducers in probe 204.

As to treatment of acne and sebaceous glands, in patients with acne it is desirable to temporarily or permanently destroy sebaceous glands. The depth at which these glands occur is approximately 1-7 mm, depending on skin thickness and body site. In accordance with various aspects of the present invention, a method and system for treating acne and sebaceous glands are provided. For example, in accordance with an exemplary embodiment, with reference to Figure 1, an exemplary treatment system 100 configured to treat a region of interest (ROI) 106 comprises a control system 102, an imaging/therapy probe with acoustic coupling 104, and display system 108.

Control system 102 and display 108 can comprise various configurations for controlling functionality of probe 104 and system 100, including for example a microprocessor with software and a plurality of input/output and communication devices, a system for controlling electronic and/or mechanical scanning and/or multiplexing of transducers, a system for power delivery, systems for monitoring, systems for sensing the spatial position of the probe and/or temporal parameters of the transducers, and/or systems for handling user input and recording treatment input and results, among others. Imaging/therapy probe 104 can comprise various probe and/or transducer configurations. For example, probe 104 can be configured for a combined dual-mode imaging/therapy transducer, coupled or co-housed imaging/therapy transducers, a separate therapy probe and separate imaging probe, or a single therapy probe. In accordance with exemplary embodiments, imaging transducers may operate at frequencies from approximately 2 to 75 MHz or more, while therapy energy can be delivered at frequencies from approximately 2 to 50 MHz, with 2 MHz to 25 MHz being typical.

With reference to Fig. 2A, an exemplary treatment method and system are configured for initially imaging a region 222 within a region of interest 206 and displaying that region 224 on a display 208 to facilitate localization of the treatment area and surrounding structures, e.g., identification of sebaceous glands 232. After localization, delivery of ultrasound energy 220 at a depth, distribution, timing, and energy level to achieve the desired therapeutic effect of thermal ablation to treat a sebaceous gland 232 is provided. Before, during, and/or after therapy, i.e., before, during and/or after delivery of ultrasound energy, monitoring of the treatment area and surrounding structures can be conducted to further planning and assessing of the results and/or providing feedback to control system 202 and a system operator.

In accordance with an exemplary embodiment, localization can be facilitated through ultrasound imaging that can be used to define the position of a sebaceous gland and/or the depth of sebaceous glands over a region of interest. Such glands can be seen lying along hair follicles and their image may be further enhanced via signal and image processing. Ultrasound imaging can also be used for safety purposes, namely, to avoid injuring vital structures. In accordance with other exemplary embodiments, localization can also be accomplished without imaging region 222, but instead can be based on prior known depths of sebaceous glands or other target regions.

For ultrasound energy delivery, probe 204 and/or imaging/therapy transducers can be mechanically and/or electronically scanned, for example along direction 226, to place treatment zones over an extended area. A treatment depth 220 can be adjusted between a range of approximately 1 to 7 mm, and/or the greatest depth of sebaceous glands 232. Such delivery of energy can occur through a repeated "image and burn" technique, i.e., imaging of the targeted sebaceous gland and then applying ultrasound energy, or through a "carpet bomb" technique, i.e., applying ultrasound energy at known depths over an extended area without initial or ongoing imaging.

With reference to Figure 2B, a treated zone 242 may extend over a line, plane, or surface, or over an extended zone across the sebaceous gland depth 240 that typically ranges from approximately 1 to 7 mm. Probe 204 can be mechanically and/or electronically scanned, for example directionally along 226, to extend treatment zone 242 over a large area. Probe 204 can be further scanned or moved along a longer directional line 228 to further enlarge treatment zone 242. For any treated zone 242, as treated zone 242 increases in depth within region of interest 206, the cross sectional area of treated zone 242 may increase in size from small to medium to large, i.e., at greater depths, the size of the treated lesion will increase. Furthermore a treated zone 242 can have a lesion shape expanding in cross section with depth, and/or be composed of the fusion of several smaller treatment zones. For example, a "cross-stitched" series of lesions, a wedge shaped series of lesions, or any suitably formed conformal lesions can be crated along treated zone 242.

The ultrasound beam from probe 204 can be spatially and/or temporally controlled by changing the spatial parameters of the transducer, such as the placement, distance, treatment depth and transducer structure, as well as by changing the temporal parameters of transducer, such as the frequency, drive amplitude, and timing, with such control handled via control system 202. Such spatial and temporal parameters can also be suitably monitored and/or utilized in open-loop and/or closed-loop feedback systems within treatment system 200. As a result of such spatial and/or temporal control, conformal lesions of various, specifically targeted, shapes, sizes and orientations can be configured along treatment zone 242.

In accordance with an exemplary embodiment, with reference to Figure 2C, one or more treated zones 242 can be configured to produce regions of heating and damage within the treatment layer in spatially defined patterns, such as a discrete locus of spaced treatment spots or two- or three- dimensional matrix of damage or destroyed tissue, e.g., a matrix of cross-stitched, ellipsoidal/cigar-shaped, wedge-shaped, mushroom-shaped or any other conformal lesions, rather than heating and destroying the entire volume of the target layer of tissue. In such a treatment where surrounding regions are spared of damage, the surrounding undamaged tissue aids rapid healing and recovery.

In accordance with another exemplary embodiment of the present invention, with reference to Figure 2D, an exemplary monitoring method may comprise monitoring the temperature profile or other tissue parameters of the region of interest 206, such as attenuation, speed of sound, or mechanical properties such as stiffness and strain of the treatment region and suitably adjust the spatial and/or temporal characteristics and energy levels of the ultrasound therapy transducer of probe 204. The results of such monitoring techniques may be indicated on display 208 by means of one-, two-, or three-dimensional images of monitoring results 250, or may simply comprise a success or fail-type indicator 252, or combinations thereof. Additional treatment monitoring techniques may be based on one or more of temperature, video, profilometry, and/or stiffness or strain gauges or any other suitable sensing technique.

In accordance with another exemplary embodiment, with reference to Figure 2E, a treatment system 200 can be configured for treatment over an expanded treatment region of interest 252 that includes a combination of tissues, such as subcutaneous fat / adipose tissue 216 and muscle 218, among others. A multiple of such tissues may be treated including sebaceous glands in combination with at least one of epidermis 212, dermis 214, adipose tissue 216, muscular fascia lying atop muscle tissue 218, mucous membrane, hair bulb 230, hair shaft 234, hair follicle between hair bulb 230 and epidermis 212, blood vessels, apocrine sweat glands, eccrine glands lying within dermis 214, fat 216 or muscle 218, and/or any other tissue of interest. For example, a treatment to region 220 of sebaceous gland 232 may be performed in combination with treatment to a region 260 of hair by suitable adjustment of the treatment spatial and/or temporal parameters of the transducers in probe 204.

As to a non-invasive method and system for the treatment of sweat gland, in accordance with an exemplary embodiment, an ultrasound transducer probe and control system are configured to deliver ultrasound energy to a targeted/specified depth and zone where the sweat gland population is required to be treated. The ultrasound beam from the transducer probe can be spatially and/or temporally adjusted, modified or otherwise controlled to match the adequate treatment of the sweat glands in the region of interest.

In accordance with exemplary embodiments, imaging transducers may operate at frequencies from approximately 2 MHz to 75 MHz or more, while therapy energy can be delivered at frequencies from approximately 500 kHz to 15 MHz, with 2 MHz to 25 MHz being typical.

With reference to Fig. 2A, sweat glands 230 are generally located within a dermis layer 214 at a depth close to hair bulbs 236. In order to treat sweat glands that require treatment in particular anatomical sites, such as, for example but not limited to, the axillary region (armpit), the palms and soles, an ultrasound transducer probe can be coupled to the skin tissue using one of the numerous coupling media, such as water, mineral oils, gels, and the like.

For example, with reference to Fig. 2B, in accordance with an exemplary embodiment an exemplary treatment method and system are configured for initially imaging a region 222 within a region of interest 206 and displaying that region 224 on a display 208 to facilitate localization of the treatment area and surrounding structures, e.g., identification of sweat glands 230. After localization, delivery of ultrasound energy 220 at a depth, distribution, timing, and energy level to achieve the desired therapeutic effect of thermal ablation to treat a sweat gland 230 is provided. Before, during, and/or after therapy, i.e., before, during and/or after delivery of ultrasound energy, monitoring of the treatment area and surrounding structures can be conducted to further planning and assessing of the results and/or providing feedback to control system 202 and a system operator.

In accordance with an exemplary embodiment, localization can be facilitated through ultrasound imaging that can be used to define the position of a sweat gland 230 and/or the depth of sweat glands 230 over a region of interest before depositing in a defined pattern at a target region 220. Such glands can be seen lying along hair follicles 232 and bulbs 236 and their image may be further enhanced via signal and image processing. Ultrasound imaging can also be used for safety purposes, namely, to avoid injuring vital structures, such as nerve endings 240. In accordance with other exemplary embodiments, localization can also be accomplished without imaging region 222, but instead can be based on prior known depths of sweat glands or other target regions, and thus be configured geometrically and/or electronically to selectively deposit energy at a particular known depth below skin surface 210 to a target region 220.

The ultrasound beam from probe 204 can be spatially and/or temporally controlled by changing the spatial parameters of the transducer, such as the placement, distance, treatment depth and transducer structure, as well as by changing the temporal parameters of transducer, such as the frequency, drive amplitude, and timing, with such control handled via control system 202. For example, in some applications, the temporal energy exposure at one location may range from approximately to 40 ms to 40 seconds, while the corresponding source frequency can suitably range from approximately 500 kHz to 15 MHz. Such spatial and temporal parameters can also be suitably monitored and/or utilized in open-loop and/or closed-loop feedback systems within treatment system 200. As a result of such spatial and/or temporal control, conformal lesions of various, specifically targeted, shapes, sizes and orientations can be configured within target region 220.

In accordance with an exemplary embodiment, the treatment resulting from ultrasound energy delivery in the region of sweat glands 230 can be used to achieve selective ablation of regions of sub-epidermal region (0.5 - 10 mm diameter zones). For example, one or more treated zones 242 can be configured to produce regions of ablative damage in spatially defined patterns, such as a discrete locus of spaced treatment spots or two- or three- dimensional matrix of damage or destroyed tissue, e.g., a matrix of cross-stitched, ellipsoidal/cigar-shaped, wedge-shaped, mushroom-shaped or any other conformal lesions, rather than heating and destroying the entire volume of the target layer of tissue. In such a treatment where surrounding regions are spared of damage, the surrounding undamaged tissue aids rapid healing and recovery.

In accordance with another exemplary embodiment, a whole contiguous sheet of treatment area can be achieved, whereby all the sweat glands within the said area are ablated. In addition to selective treatment of sweat gland regions, in accordance with another exemplary embodiment, treatment system 200 could be configured to "carpet bomb" the fat layer at 1-7 mm depth, e.g., up to 90% of the sweat glands in the armpit can be ablated without any physiologic issues.

In accordance with another exemplary embodiment of the present invention, an exemplary monitoring method may comprise monitoring the temperature profile or other tissue parameters of the region of interest 206, such as attenuation, speed of sound, or mechanical properties such as stiffness and strain of the treatment region and suitably adjust the spatial and/or temporal characteristics and energy levels of the ultrasound therapy transducer of probe 204. The results of such monitoring techniques may be indicated on display 208 by means of one-, two-, or three-dimensional images of monitoring results 250, or may simply comprise a success or fail-type indicator 252, or combinations thereof. Additional treatment monitoring techniques may be based on one or more of temperature, video, profilometry, and/or stiffness or strain gauges or any other suitable sensing technique. The non-thermal effects from an acoustic field can also "shock" the sweat producing apocrine and eccrine cells in to reduced activity. These effects mentioned here as examples are, but not limited to, acoustic cavitation, acoustic streaming, inter-cellular shear effects, cell resonant effects, and the like.

In accordance with an exemplary embodiment, focused or directive ultrasound energy can be used for the treatment of sweat glands in the armpit (without the combination of pharmacological formulations). For example, a clinical indication would be to use in the management of *Hidradenitis suppurativa*. Ultrasound energy deposited at a selective depth can also be used in combination with a number of pharmaceutical formulations that are currently prescribed for the treatment of sweat gland hyperactivity in the axillary region, palms and soles. The ultrasound energy delivered to the target region in combination with the pharmaceutical agents such as BOTOX^{®} or retinoids can help synergistically treat the sweat gland region by, (1) increasing activity of the agents due to the thermal and non-thermal mechanisms, (2) reduced requirement of overall drug dosage, as well as reducing the drug toxicity, (3) increase local effect of drug in a site selective manner.

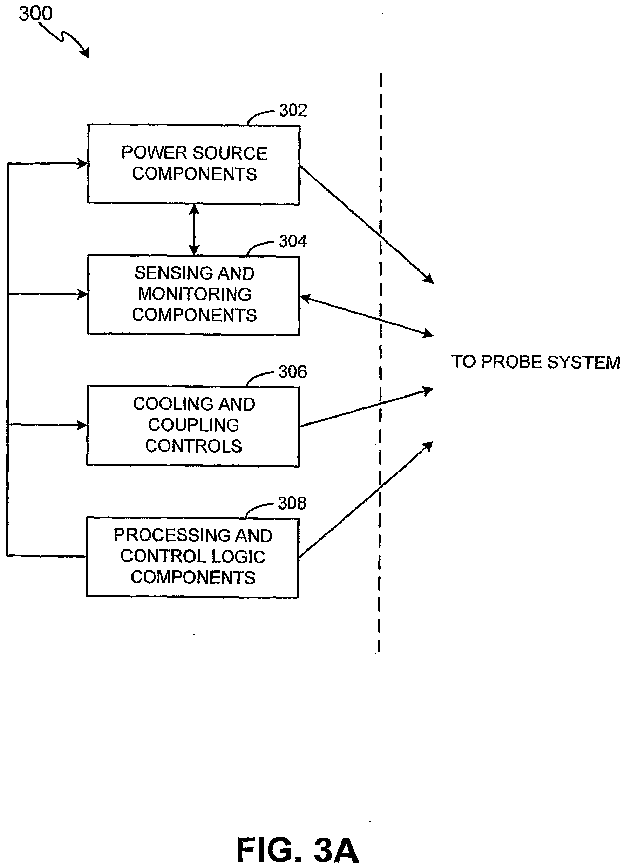

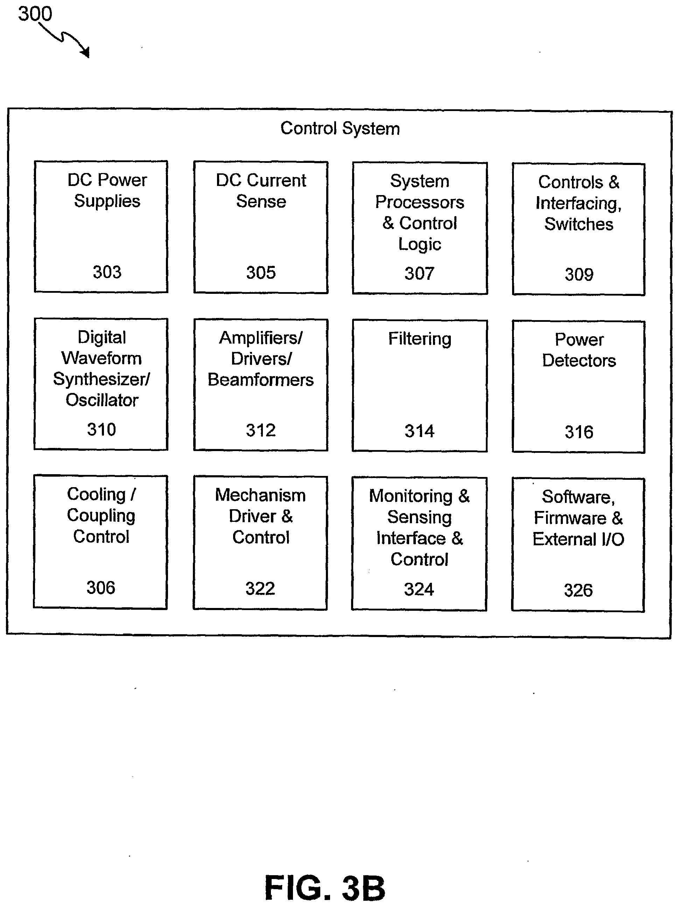

An exemplary control system 202 and display system 208 may be configured in various manners for controlling probe and system functionality. With reference to FIGS. 3A and 3B, in accordance with exemplary embodiments, an exemplary control system 300 can be configured for coordination and control of the entire therapeutic treatment process for tissue treatment. For example, control system 300 can suitably comprise power source components 302, sensing and monitoring components 304, cooling and coupling controls 306, and/or processing and control logic components 308. Control system 300 can be configured and optimized in a variety of ways with more or less subsystems and components to implement the therapeutic system for tissue treatment, and the embodiments in FIGS. 3A and 3B are merely for illustration purposes.

For example, for power sourcing components 302, control system 300 can comprise one or more direct current (DC) power supplies 303 configured to provide electrical energy for entire control system 300, including power required by a transducer electronic amplifier/driver 312. A DC current sense device 305 can also be provided to confirm the level of power going into amplifiers/drivers 312 for safety and monitoring purposes.

Amplifiers/drivers 312 can comprise multi-channel or single channel power amplifiers and/or drivers. In accordance with an exemplary embodiment for transducer array configurations, amplifiers/drivers 312 can also be configured with a beamformer to facilitate array focusing. An exemplary beamformer can be electrically excited by an oscillator/digitally controlled waveform synthesizer 310 with related switching logic.

The power sourcing components can also include various filtering configurations 314. For example, switchable harmonic filters and/or matching may be used at the output of amplifier/driver 312 to increase the drive efficiency and effectiveness. Power detection components 316 may also be included to confirm appropriate operation and calibration. For example, electric power and other energy detection components 316 may be used to monitor the amount of power going to an exemplary probe system.

Various sensing and monitoring components 304 may also be suitably implemented within control system 300. For example, in accordance with an exemplary embodiment, monitoring, sensing and interface control components 324 may be configured to operate with various motion detection systems implemented within transducer probe 204 to receive and process information such as acoustic or other spatial and temporal information from a region of interest. Sensing and monitoring components can also include various controls, interfacing and switches 309 and/or power detectors 316. Such sensing and monitoring components 304 can facilitate open-loop and/or closed-loop feedback systems within treatment system 200.

Cooling/coupling control systems 306 may be provided to remove waste heat from an exemplary probe 204, provide a controlled temperature at the superficial tissue interface and deeper into tissue, and/or provide acoustic coupling from transducer probe 204 to region-of-interest 206. Such cooling/coupling control systems 306 can also be configured to operate in both open-loop and/or closed-loop feedback arrangements with various coupling and feedback components.

Processing and control logic components 308 can comprise various system processors and digital control logic 307, such as one or more of microcontrollers, microprocessors, field-programmable gate arrays (FPGAs), computer boards, and associated components, including firmware and control software 326, which interfaces to user controls and interfacing circuits as well as input/output circuits and systems for communications, displays, interfacing, storage, documentation, and other useful functions. System software and firmware 326 controls all initialization, timing, level setting, monitoring, safety monitoring, and all other system functions required to accomplish user-defined treatment objectives. Further, various control switches 308 can also be suitably configured to control operation.

An exemplary transducer probe 204 can also be configured in various manners and comprise a number of reusable and/or disposable components and parts in various embodiments to facilitate its operation. For example, transducer probe 204 can be configured within any type of transducer probe housing or arrangement for facilitating the coupling of transducer to a tissue interface, with such housing comprising various shapes, contours and configurations. Transducer probe 204 can comprise any type of matching, such as for example, electric matching, which may be electrically switchable; multiplexer circuits and/or aperture/element selection circuits; and/or probe identification devices, to certify probe handle, electric matching, transducer usage history and calibration, such as one or more serial EEPROM (memories). Transducer probe 204 may also comprise cables and connectors; motion mechanisms, motion sensors and encoders; thermal monitoring sensors; and/or user control and status related switches, and indicators such as LEDs. For example, a motion mechanism in probe 204 may be used to controllably create multiple lesions, or sensing of probe motion itself may be used to controllably create multiple lesions and/or stop creation of lesions, e.g. for safety reasons if probe 204 is suddenly jerked or is dropped. In addition, an external motion encoder arm may be used to hold the probe during use, whereby the spatial position and attitude of probe 104 is sent to the control system to help controllably create lesions. Furthermore, other sensing functionality such as profilometers or other imaging modalities may be integrated into the probe in accordance with various exemplary embodiments. Moreover, the therapy contemplated herein can also be produced, for example, by transducers disclosed in U.S. Application Serial No. 10/944,499, filed on September 16, 2004, entitled METHOD AND SYSTEM FOR ULTRASOUND TREATMENT WITH A MULTI-DIRECTIONAL TRANSDUCER, published as US 2006/0058717 A1, and U.S. Application Serial No. 10/944,500, filed on September 16, 2004, and entitled SYSTEM AND METHOD FOR VARIABLE DEPTH ULTRASOUND TREATMENT, published as US 2006/0058664 A1.

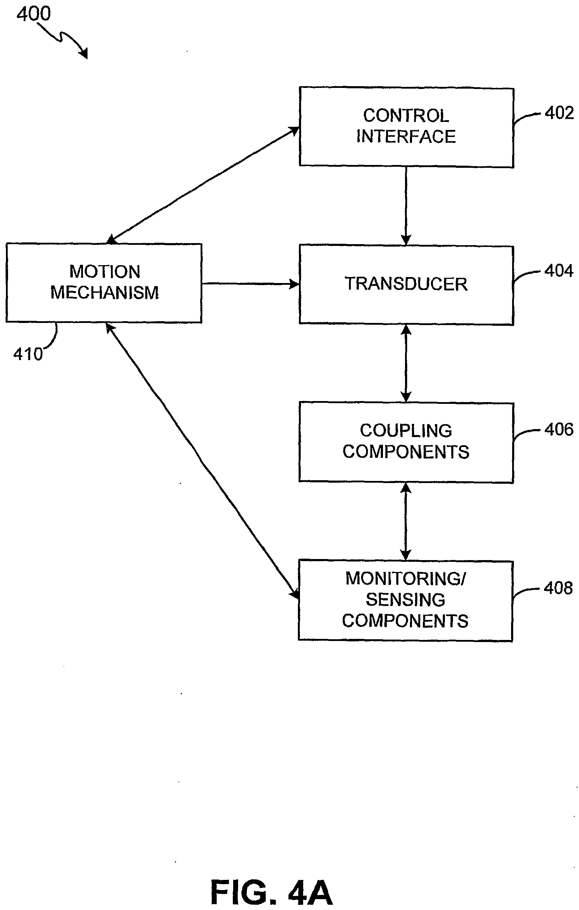

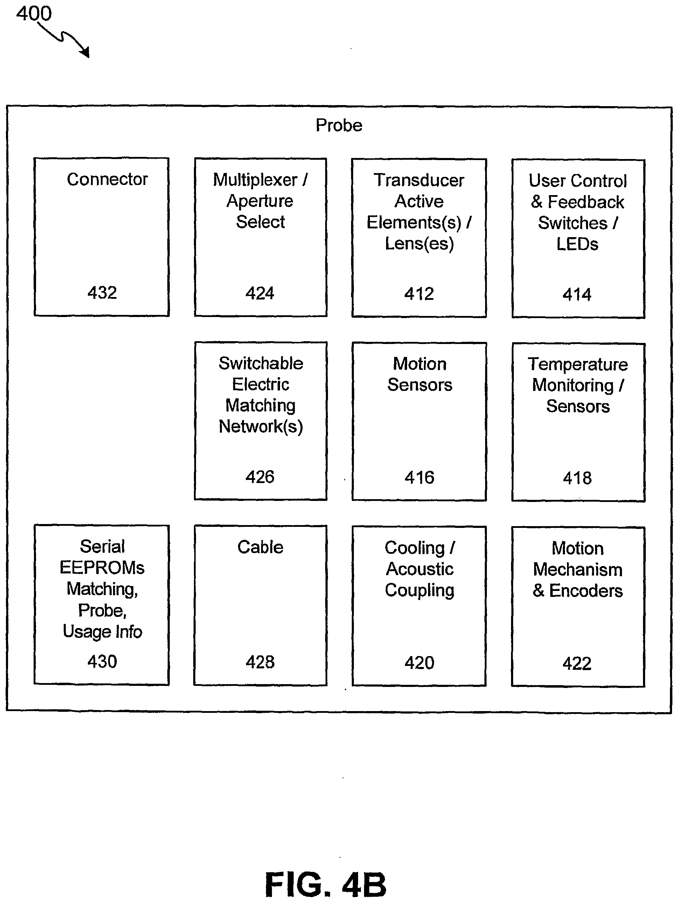

With reference to FIGS. 4A and 4B, in accordance with an exemplary embodiment, a transducer probe 400 can comprise a control interface 402, a transducer 404, coupling components 406, and monitoring/sensing components 408, and/or motion mechanism 410. However, transducer probe 400 can be configured and optimized in a variety of ways with more or less parts and components to provide ultrasound energy for controlled thermal injury, and the embodiment in FIGS. 4A and 4B are merely for illustration purposes.

Control interface 402 is configured for interfacing with control system 300 to facilitate control of transducer probe 400. Control interface components 402 can comprise multiplexer/aperture select 424, switchable electric matching networks 426, serial EEPROMs and/or other processing components and matching and probe usage information 430 and interface connectors 432.

Coupling components 406 can comprise various devices to facilitate coupling of transducer probe 400 to a region of interest. For example, coupling components 406 can comprise cooling and acoustic coupling system 420 configured for acoustic coupling of ultrasound energy and signals. Acoustic cooling/coupling system 420 with possible connections such as manifolds may be utilized to couple sound into the region-of-interest, control temperature at the interface and deeper into tissue, provide liquid-filled lens focusing, and/or to remove transducer waste heat. Coupling system 420 may facilitate such coupling through use of various coupling mediums, including air and other gases, water and other fluids, gels, solids, and/or any combination thereof, or any other medium that allows for signals to be transmitted between transducer active elements 412 and a region of interest. In addition to providing a coupling function, in accordance with an exemplary embodiment, coupling system 420 can also be configured for providing temperature control during the treatment application. For example, coupling system 420 can be configured for controlled cooling of an interface surface or region between transducer probe 400 and a region of interest and beyond by suitably controlling the temperature of the coupling medium. The suitable temperature for such coupling medium can be achieved in various manners, and utilize various feedback systems, such as thermocouples, thermistors or any other device or system configured for temperature measurement of a coupling medium. Such controlled cooling can be configured to further facilitate spatial and/or thermal energy control of transducer probe 400.

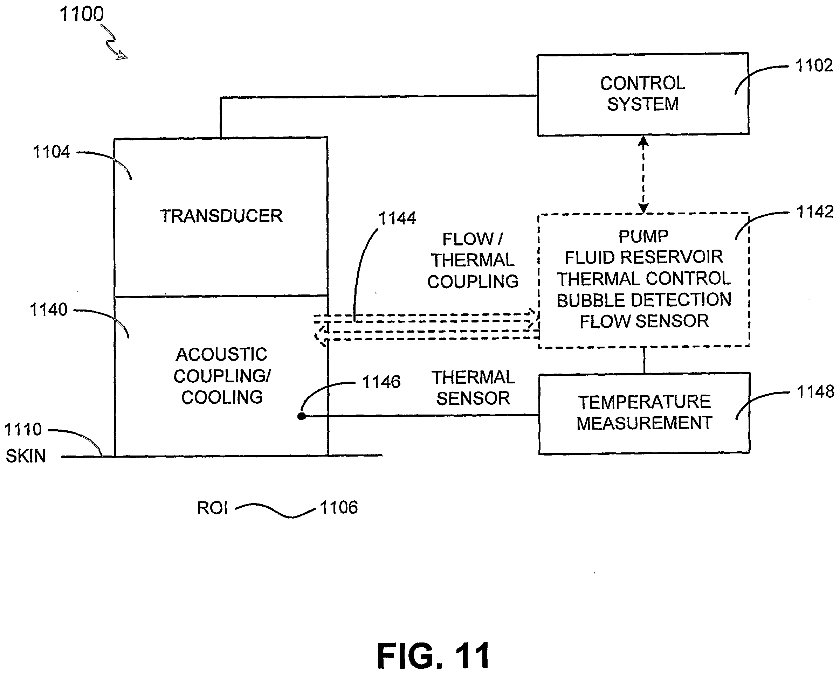

In accordance with an exemplary embodiment, with additional reference to Fig. 11, acoustic coupling and cooling 1140 can be provided to acoustically couple energy and imaging signals from transducer probe 1104 to and from the region of interest 1106, to provide thermal control at the probe to region-of-interest interface 1110 and deeper into tissue, and to remove potential waste heat from the transducer probe at region 1144. Temperature monitoring can be provided at the coupling interface via a thermal sensor 1146 to provides a mechanism of temperature measurement 1148 and control via control system 1102 and a thermal control system 1142. Thermal control may consist of passive cooling such as via heat sinks or natural conduction and convection or via active cooling such as with peltier thermoelectric coolers, refrigerants, or fluid-based systems comprised of pump, fluid reservoir, bubble detection, flow sensor, flow channels/tubing 1144 and thermal control 1142.

With continued reference to Fig. 4, monitoring and sensing components 408 can comprise various motion and/or position sensors 416, temperature monitoring sensors 418, user control and feedback switches 414 and other like components for facilitating control by control system 300, e.g., to facilitate spatial and/or temporal control through open-loop and closed-loop feedback arrangements that monitor various spatial and temporal characteristics.

Motion mechanism 410 can comprise manual operation, mechanical arrangements, or some combination thereof. For example, a motion mechanism 422 can be suitably controlled by control system 300, such as through the use of accelerometers, encoders or other position/orientation devices 416 to determine and enable movement and positions of transducer probe 400. Linear, rotational or variable movement can be facilitated, e.g., those depending on the treatment application and tissue contour surface.

Transducer 404 can comprise one or more transducers configured for treating of SMAS layers and targeted regions. Transducer 404 can also comprise one or more transduction elements and/or lenses 412. The transduction elements can comprise a piezoelectrically active material, such as lead zirconante titanate (PZT), or any other piezoelectrically active material, such as a piezoelectric ceramic, crystal, plastic, and/or composite materials, as well as lithium niobate, lead titanate, barium titanate, and/or lead metaniobate. In addition to, or instead of, a piezoelectrically active material, transducer 404 can comprise any other materials configured for generating radiation and/or acoustical energy. Transducer 404 can also comprise one or more matching layers configured along with the transduction element such as coupled to the piezoelectrically active material. Acoustic matching layers and/or damping may be employed as necessary to achieve the desired electroacoustic response.

In accordance with an exemplary embodiment, the thickness of the transduction element of transducer 404 can be configured to be uniform. That is, a transduction element 412 can be configured to have a thickness that is substantially the same throughout. In accordance with another exemplary embodiment, the thickness of a transduction element 412 can also be configured to be variable. For example, transduction element(s) 412 of transducer 404 can be configured to have a first thickness selected to provide a center operating frequency of approximately 2 MHz to 75 MHz, such as for imaging applications.
Transduction element 412 can also be configured with a second thickness selected to provide a center operating frequency of approximately 2 to 400 MHz, and typically between 4 MHz and 15 MHz for therapy application. Transducer 404 can be configured as a single broadband transducer excited with at least two or more frequencies to provide an adequate output for generating a desired response. Transducer 404 can also be configured as two or more individual transducers, wherein each transducer comprises one or more transduction element. The thickness of the transduction elements can be configured to provide center-operating frequencies in a desired treatment range.

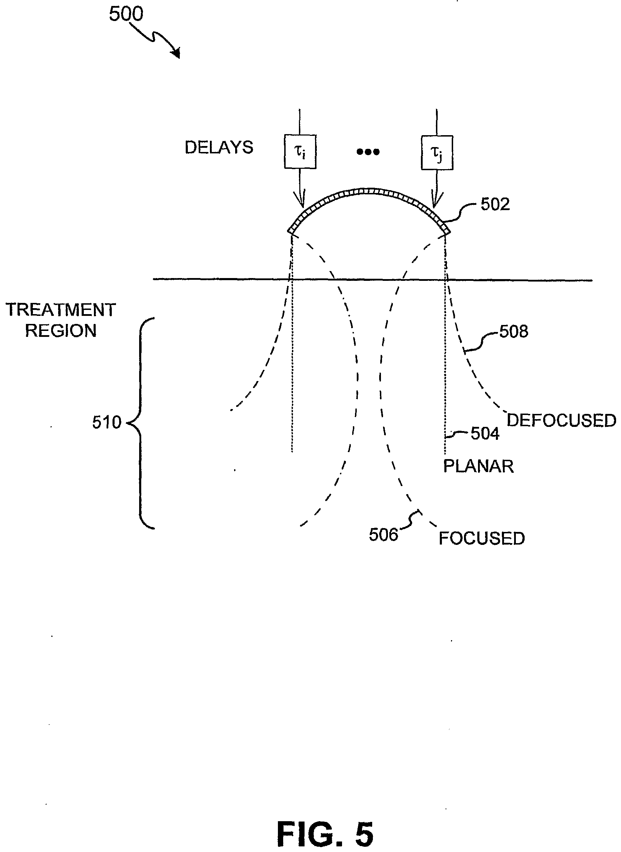

Transducer 404 may be composed of one or more individual transducers in any combination of focused, planar, or unfocused single-element, multi-element, or array transducers, including 1-D, 2-D, and annular arrays; linear, curvilinear, sector, or spherical arrays; spherically, cylindrically, and/or electronically focused, defocused, and/or lensed sources. For example, with reference to an exemplary embodiment depicted in Fig. 5, transducer 500 can be configured as an acoustic array to facilitate phase focusing. That is, transducer 500 can be configured as an array of electronic apertures that may be operated by a variety of phases via variable electronic time delays. By the term "operated," the electronic apertures of transducer 500 may be manipulated, driven, used, and/or configured to produce and/or deliver an energy beam corresponding to the phase variation caused by the electronic time delay. For example, these phase variations can be used to deliver defocused beams, planar beams, and/or focused beams, each of which may be used in combination to achieve different physiological effects in a region of interest 510. Transducer 500 may additionally comprise any software and/or other hardware for generating, producing and or driving a phased aperture array with one or more electronic time delays.

Transducer 500 can also be configured to provide focused treatment to one or more regions of interest using various frequencies. In order to provide focused treatment, transducer 500 can be configured with one or more variable depth devices to facilitate treatment. For example, transducer 500 may be configured with variable depth devices disclosed in U.S. Patent Application 10/944, 500. In addition, transducer 500 can also be configured to treat one or more additional ROI 510 through the enabling of sub-harmonics or pulse-echo imaging, as disclosed in U.S. Patent Application 10/944,499.

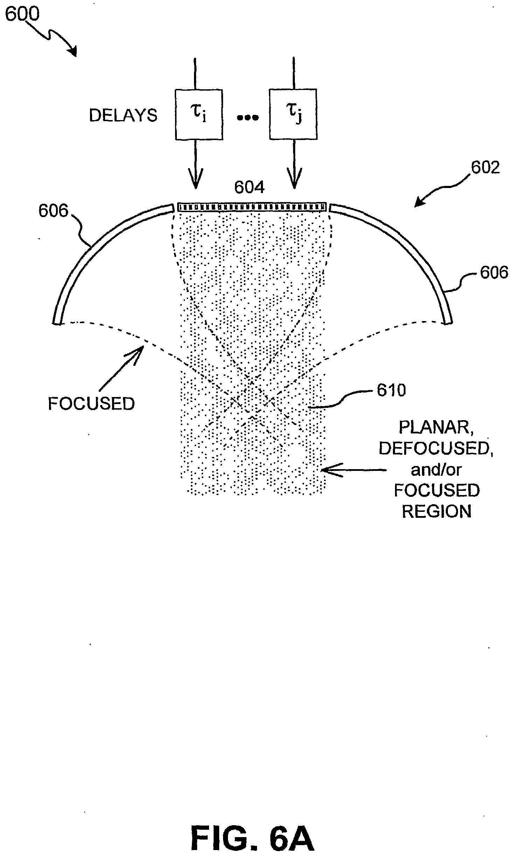

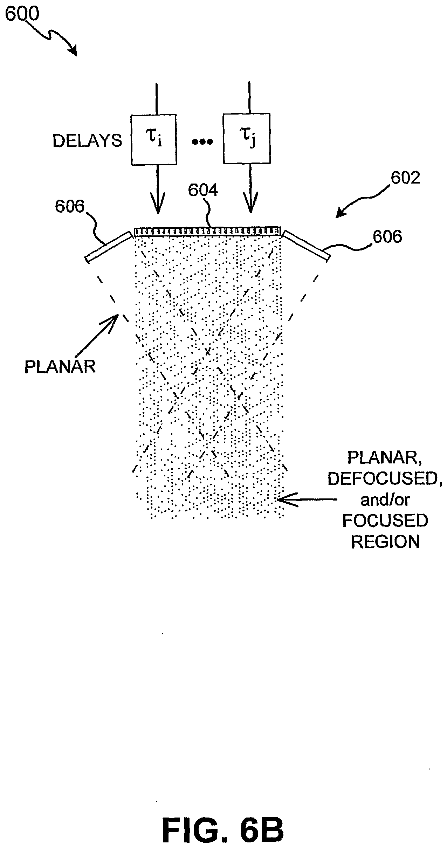

Moreover, any variety of mechanical lenses or variable focus lenses, e.g. liquid-filled lenses, may also be used to focus and or defocus the sound field. For example, with reference to exemplary embodiments depicted in Figs. 6A and 6B, transducer 600 may also be configured with an electronic focusing array 604 in combination with one or more transduction elements 606 to facilitate increased flexibility in treating ROI 610. Array 604 may be configured in a manner similar to transducer 502. That is, array 604 can be configured as an array of electronic apertures that may be operated by a variety of phases via variable electronic time delays, for example, T₁, T₂...Tⱼ. By the term "operated," the electronic apertures of array 604 may be manipulated, driven, used, and/or configured to produce and/or deliver energy in a manner corresponding to the phase variation caused by the electronic time delay. For example, these phase variations can be used to deliver defocused beams, planar beams, and/or focused beams, each of which may be used in combination to achieve different physiological effects in ROI 610.

Transduction elements 606 may be configured to be concave, convex, and/or planar. For example, in an exemplary embodiment depicted in Fig. 6A, transduction elements 606A are configured to be concave in order to provide focused energy for treatment of ROI 610. Additional embodiments are disclosed in U.S. Patent Application 10/944,500.

In another exemplary embodiment, depicted in Fig. 6B, transduction elements 606B can be configured to be substantially flat in order to provide substantially uniform energy to ROI 610. While Figs. 6A and 6B depict exemplary embodiments with transduction elements 604 configured as concave and substantially flat, respectively, transduction elements 604 can be configured to be concave, convex, and/or substantially flat. In addition, transduction elements 604 can be configured to be any combination of concave, convex, and/or substantially flat structures. For example, a first transduction element can be configured to be concave, while a second transduction element can be configured to be substantially flat.

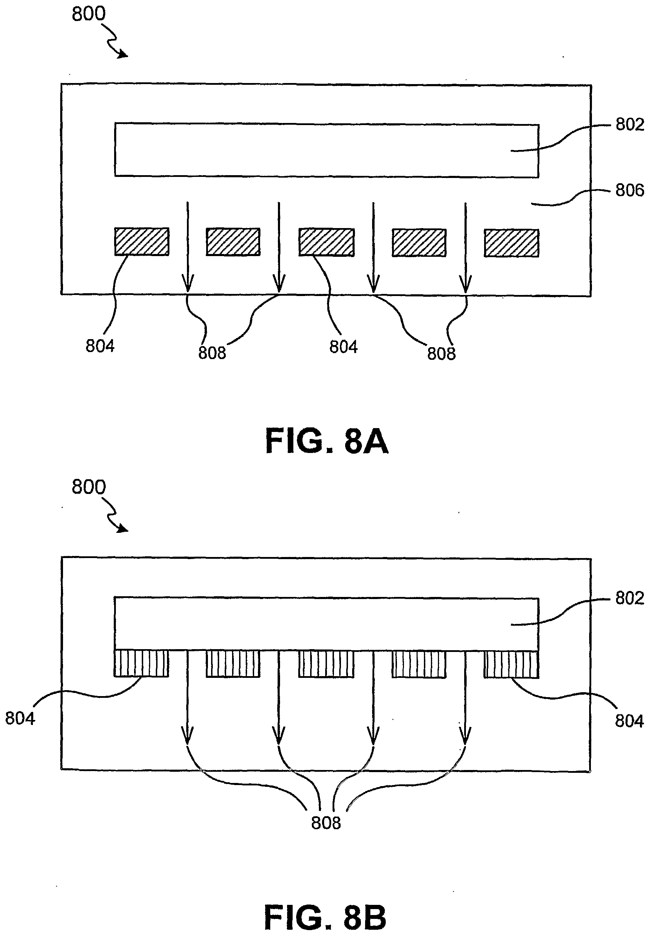

With reference to Figs. 8A and 8B, transducer 404 can be configured as single-element arrays, wherein a single-element 802, e.g., a transduction element of various structures and materials, can be configured with a plurality of masks 804, such masks comprising ceramic, metal or any other material or structure for masking or altering energy distribution from element 802, creating an array of energy distributions 808. Masks 804 can be coupled directly to element 802 or separated by a standoff 806, such as any suitably solid or liquid material.

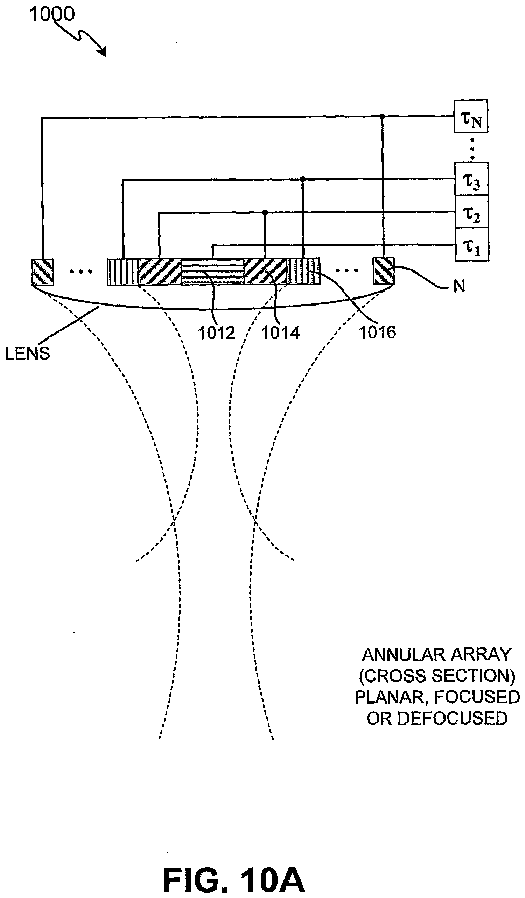

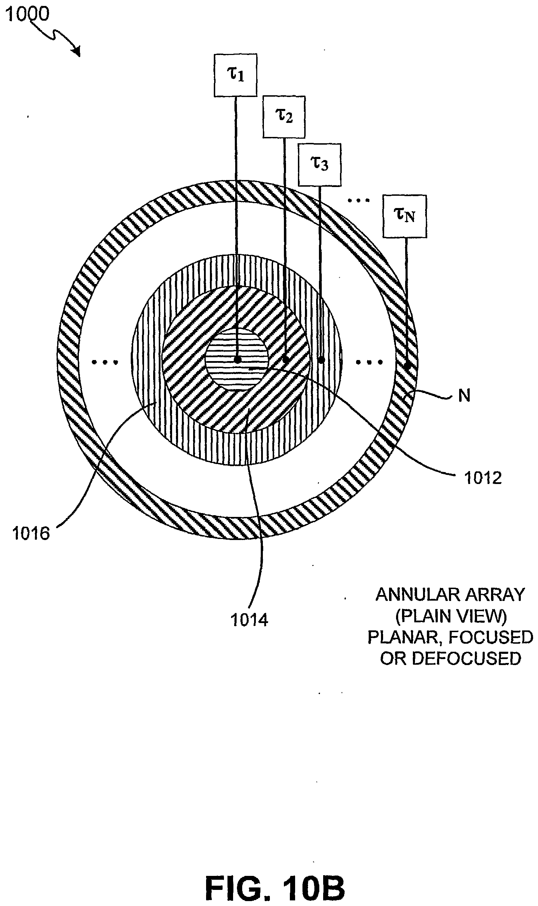

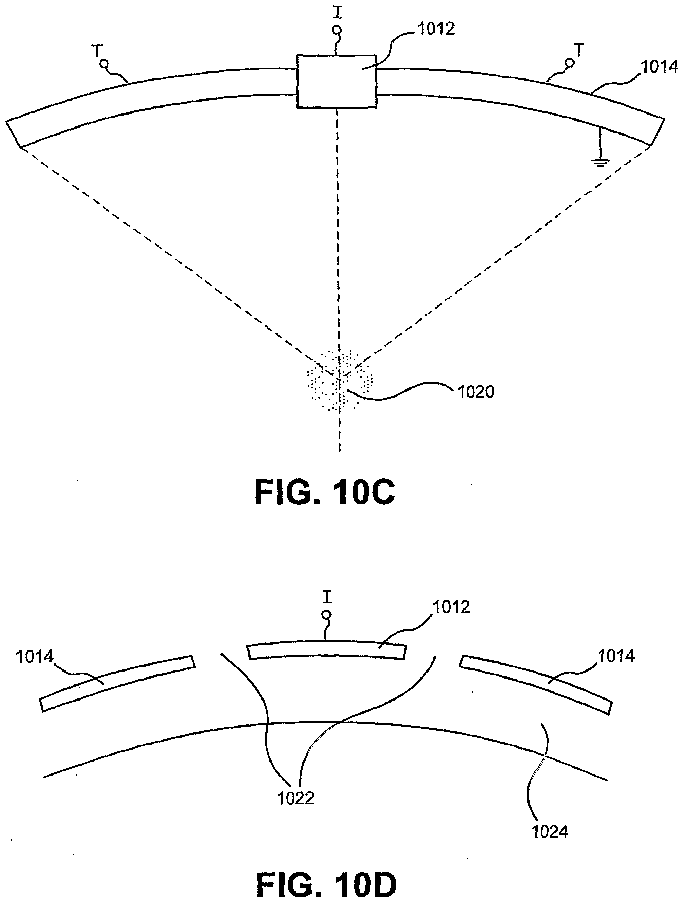

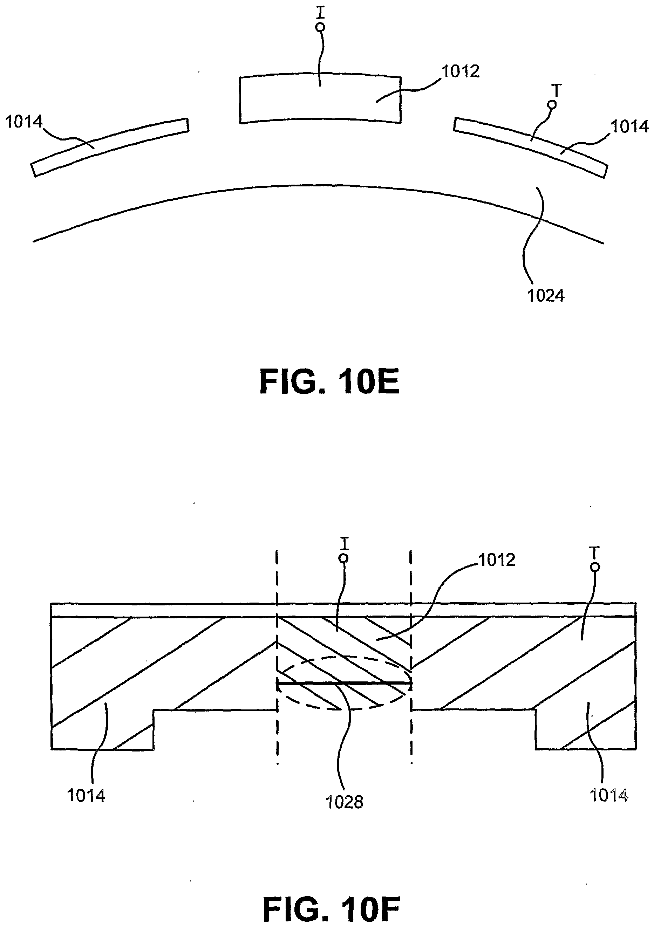

An exemplary transducer 404 can also be configured as an annular array to provide planar, focused and/or defocused acoustical energy. For example, with reference to Figs. 10A and 10B, in accordance with an exemplary embodiment, an annular array 1000 can comprise a plurality of rings 1012, 1014, 1016 to N. Rings 1012, 1014, 1016 to N can be mechanically and electrically isolated into a set of individual elements, and can create planar, focused, or defocused waves. For example, such waves can be centered on-axis, such as by methods of adjusting corresponding transmit and/or receive delays, τ₁, τ₂, τ₃ ... τ_{N}. An electronic focus can be suitably moved along various depth positions, and can enable variable strength or beam tightness, while an electronic defocus can have varying amounts of defocusing. In accordance with an exemplary embodiment, a lens and/or convex or concave shaped annular array 1000 can also be provided to aid focusing or defocusing such that any time differential delays can be reduced. Movement of annular array 800 in one, two or three-dimensions, or along any path, such as through use of probes and/or any conventional robotic arm mechanisms, may be implemented to scan and/or treat a volume or any corresponding space within a region of interest.

Transducer 404 can also be configured in other annular or non-array configurations for imaging/therapy functions. For example, with reference to Figs. 10C-10F, a transducer can comprise an imaging element 1012 configured with therapy element(s) 1014. Elements 1012 and 1014 can comprise a single-transduction element, e.g., a combined imaging/transducer element, or separate elements, can be electrically isolated 1022 within the same transduction element or between separate imaging and therapy elements, and/or can comprise standoff 1024 or other matching layers, or any combination thereof. For example, with particular reference to Fig. 10F, a transducer can comprise an imaging element 1012 having a surface 1028 configured for focusing, defocusing or planar energy distribution, with therapy elements 1014 including a stepped-configuration lens configured for focusing, defocusing, or planar energy distribution.

In accordance with various exemplary embodiments, transducer 404 may be configured to provide one, two and/or three-dimensional treatment applications for focusing acoustic energy to one or more regions of interest. For example, as discussed above, transducer 404 can be suitably diced to form a one-dimensional array, e.g., transducer 602 comprising a single array of sub-transduction elements.

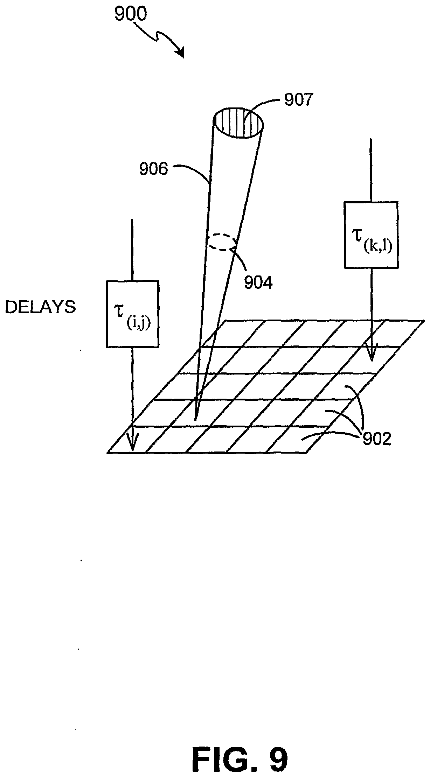

In accordance with another exemplary embodiment, transducer 404 may be suitably diced in two-dimensions to form a two-dimensional array. For example, with reference to Fig. 9, an exemplary two-dimensional array 900 can be suitably diced into a plurality of two-dimensional portions 902. Two-dimensional portions 902 can be suitably configured to focus on the treatment region at a certain depth, and thus provide respective slices 904 of the treatment region. As a result, the two-dimensional array 900 can provide a two-dimensional slicing of the image place of a treatment region, thus providing two-dimensional treatment.

In accordance with another exemplary embodiment, transducer 404 may be suitably configured to provide three-dimensional treatment. For example, to provide-three dimensional treatment of a region of interest, with reference again to Fig. 1, a three-dimensional system can comprise a transducer within probe 104 configured with an adaptive algorithm, such as, for example, one utilizing three-dimensional graphic software, contained in a control system, such as control system 102. The adaptive algorithm is suitably configured to receive two-dimensional imaging, temperature and/or treatment or other tissue parameter information relating to the region of interest, process the received information, and then provide corresponding three-dimensional imaging, temperature and/or treatment information.

In accordance with an exemplary embodiment, with reference again to Fig. 9, an exemplary three-dimensional system can comprise a two-dimensional array 900 configured with an adaptive algorithm to suitably receive 904 slices from different image planes of the treatment region, process the received information, and then provide volumetric information 906, e.g., three-dimensional imaging, temperature and/or treatment information. Moreover, after processing the received information with the adaptive algorithm, the two-dimensional array 900 may suitably provide therapeutic heating to the volumetric region 906 as desired. In accordance with other exemplary embodiments, rather than utilizing an adaptive algorithm, such as three-dimensional software, to provide three-dimensional imaging and/or temperature information, an exemplary three-dimensional system can comprise a single transducer 404 configured within a probe arrangement to operate from various rotational and/or translational positions relative to a target region.

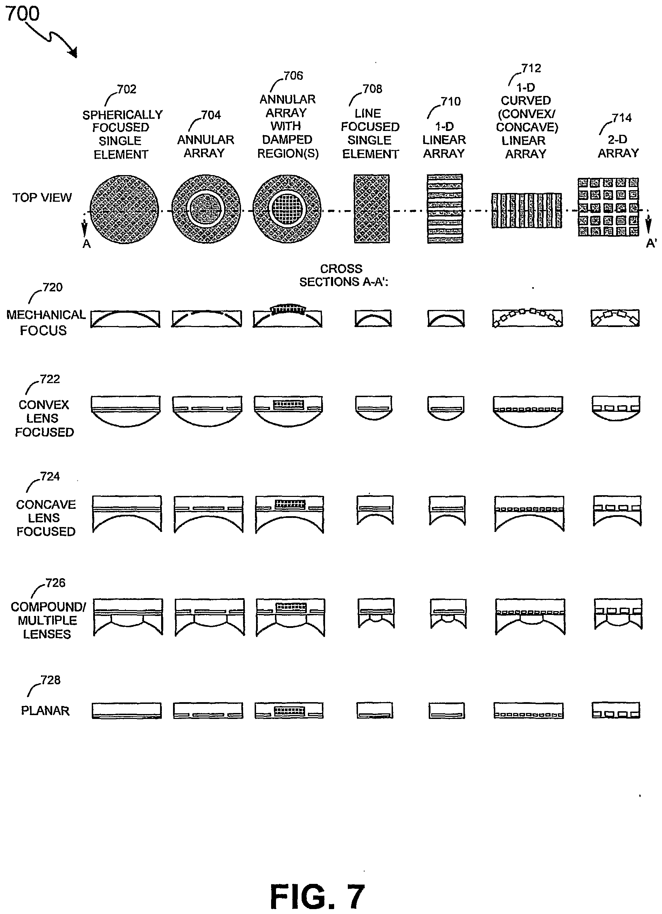

To further illustrate the various structures for transducer 404, with reference to Figure 7, ultrasound therapy transducer 700 can be configured for a single focus, an array of foci, a locus of foci, a line focus, and/or diffraction patterns. Transducer 700 can also comprise single elements, multiple elements, annular arrays, one-, two-, or three-dimensional arrays, broadband transducers, and/or combinations thereof, with or without lenses, acoustic components, and mechanical and/or electronic focusing. Transducers configured as spherically focused single elements 702, annular arrays 704, annular arrays with damped regions 706, line focused single elements 708, 1-D linear arrays 710, 1-D curvilinear arrays in concave or convex form, with or without elevation focusing, 2-D arrays, and 3-D spatial arrangements of transducers may be used to perform therapy and/or imaging and acoustic monitoring functions. For any transducer configuration, focusing and/or defocusing may be in one plane or two planes via mechanical focus 720, convex lens 722, concave lens 724, compound or multiple lenses 726, planar form 728, or stepped form, such as illustrated in Fig, 10F. Any transducer or combination of transducers may be utilized for treatment. For example, an annular transducer may be used with an outer portion dedicated to therapy and the inner disk dedicated to broadband imaging wherein such imaging transducer and therapy transducer have different acoustic lenses and design, such as illustrated in Fig. 10C-10F.

Moreover, such transduction elements 700 may comprise a piezoelectrically active material, such as lead zirconante titanate (PZT), or any other piezoelectrically active material, such as a piezoelectric ceramic, crystal, plastic, and/or composite materials, as well as lithium niobate, lead titanate, barium titanate, and/or lead metaniobate. Transduction elements 700 may also comprise one or more matching layers configured along with the piezoelectrically active material. In addition to or instead of piezoelectrically active material, transduction elements 700 can comprise any other materials configured for generating radiation and/or acoustical energy. A means of transferring energy to and from the transducer to the region of interest is provided.

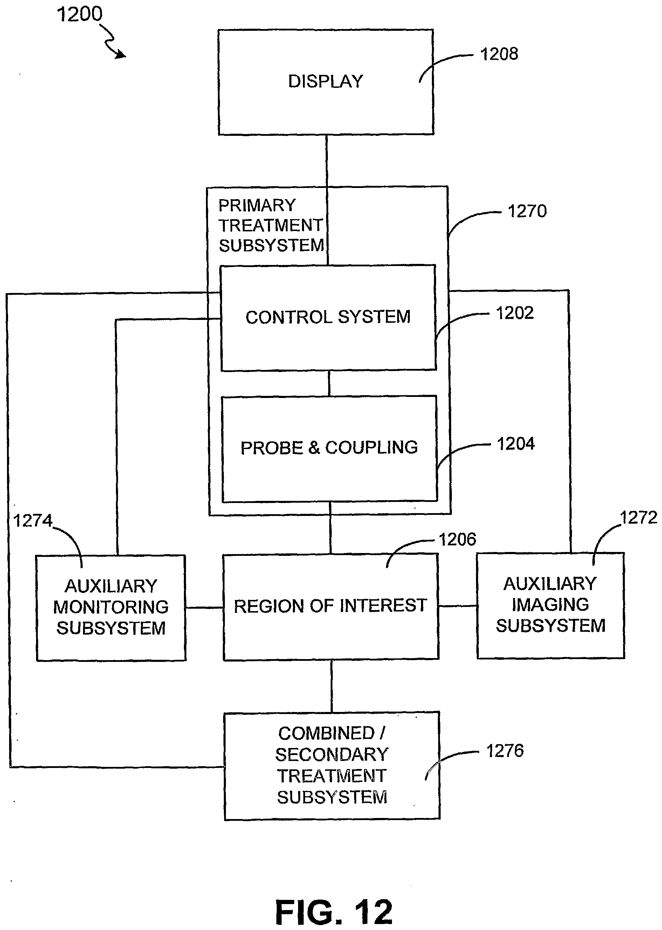

In accordance with another exemplary embodiment, with reference to Fig. 12, an exemplary treatment system 200 can be configured with and/or combined with various auxiliary systems to provide additional functions. For example, an exemplary treatment system 1200 for treating a region of interest 1206 can comprise a control system 1202, a probe 1204, and a display 1208. Treatment system 1200 further comprises an auxiliary imaging modality 1272 and/or auxiliary monitoring modality 1274 may be based upon at least one of photography and other visual optical methods, magnetic resonance imaging (MRI), computed tomography (CT), optical coherence tomography (OCT), electromagnetic, microwave, or radio frequency (RF) methods, positron emission tomography (PET), infrared, ultrasound, acoustic, or any other suitable method of visualization, localization, or monitoring of SMAS layers within region-of-interest 1206, including imaging/monitoring enhancements. Such imaging/monitoring enhancement for ultrasound imaging via probe 1204 and control system 1202 could comprise M-mode, persistence, filtering, color, Doppler, and harmonic imaging among others; furthermore an ultrasound treatment system 1270, as a primary source of treatment, may be combined with a secondary source of treatment 1276, including radio frequency (RF), intense pulsed light (IPL), laser, infrared laser, microwave, or any other suitable energy source.

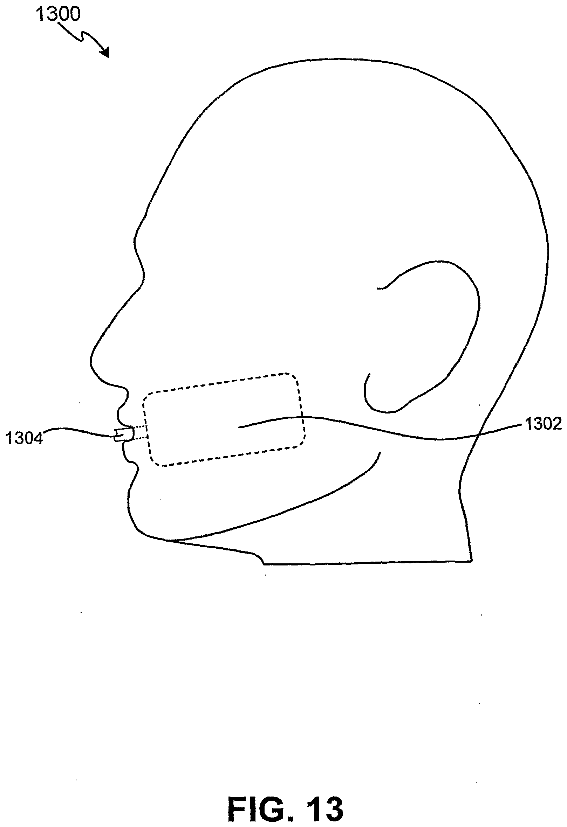

In accordance with another exemplary embodiment, with reference to Figure 13, treatment composed of imaging, monitoring, and/or therapy to a region of interest may be further aided, augmented, and/or delivered with passive or active devices 1304 within the oral cavity. For example, if passive or active device 1304 is a second transducer or acoustic reflector acoustically coupled to the cheek lining it is possible to obtain through transmission, tomographic, or round-trip acoustic waves which are useful for treatment monitoring, such as in measuring acoustic speed of sound and attenuation, which are temperature dependent; furthermore such a transducer could be used to treat and/or image. In addition an active, passive, or active/passive object 1304 may be used to flatten the skin, and/or may be used as an imaging grid, marker, or beacon, to aid determination of position. A passive or active device 1304 may also be used to aid cooling or temperature control. Natural air in the oral cavity may also be used as passive device 1304 whereby it may be utilized to as an acoustic reflector to aid thickness measurement and monitoring function.

## Claims

1. An ultrasound treatment system configured for noninvasive face lifts and deep tissue tightening, said ultrasound treatment system comprising:
a probe system (204) configured for both imaging and therapy, wherein said probe system comprises an imaging and therapy transducer configured for targeted delivery of ablative ultrasound within the superficial muscular aponeurosis system (SMAS) layer, and wherein the imaging and therapy transducer comprises an imaging element and a single therapy element,
wherein the imaging element is configured to image a region of interest (206) comprising the SMAS layer (216),
wherein the single therapy element is composed of one individual single-element transducer, configured for single focus and for targeted delivery of ablative ultrasound energy (220) into said SMAS layer (216) at a depth of up to 15mm below the skin surface for thermal injury of tissue and to cause shrinkage of said SMAS layer (216);
a control system (202) coupled to said probe system (204), said control system (202) controlling mechanical scanning of the imaging and therapy transducer and comprising a spatial control and a temporal control, said spatial control and said temporal control controlling said targeted delivery of ablative ultrasound energy (220) to cause said shrinkage of said at least a portion of said SMAS layer (216);
a motion mechanism for controllably creating multiple lesions, wherein the imaging and therapy transducer within the probe system (204) is mechanically scanned in a direction (226) to place treatment zones (260) over an extended area formed as a line; and
a display system (208) coupled to said control system (202) and to said imaging element of said probe system (204), said display system (208) configured for displaying images (224) of said region of interest (206).

2. The ultrasound treatment system according to claim 1, wherein the imaging element has a first thickness, wherein the single therapy element has a second thickness, and wherein the imaging element is configured to image with an imaging frequency of between 2 MHz to 75 MHz and the single therapy element is configured
to treat with a treatment frequency of between 4MHz and 15MHz.

3. The ultrasound treatment system according to any one of the preceding claims, wherein the control system comprises a processor configured to execute an algorithm operable to convert data from the probe system and to display a three dimensional image of said region of interest on said display system (208).

4. The ultrasound treatment system according to any one of the preceding claims, wherein the motion mechanism comprises at least one of the group consisting of an accelerometer, encoder and a position/orientation device.

5. The ultrasound treatment system according to any one of the preceding claims, wherein the imaging element and the therapy element are integrated into a single dual- function imaging/therapy transducer element.

6. The ultrasound treatment system according to any one of the preceding claims, further comprising an auxiliary imaging apparatus (1272), a treatment monitoring apparatus (1274) and a secondary therapy system (1276).

7. The ultrasound treatment system according to claim 6, wherein said auxiliary imaging apparatus (1272) comprises at least one of a photographic device and an optical modality.

8. The ultrasound treatment system according to any one of the preceding claims, wherein said probe system (204) is configured for targeted delivery of said ablative ultrasound energy (220) to thermally injure a portion of at least one of skin (210), dermis (212), muscular fascia (216), and adipose (214) in addition to said SMAS layer (216) for destruction of tissue and to cause shrinkage of said SMAS layer (216) for any one of a face lift, treatment of photoaged tissue, treatment of excessive sweating, treatment of hyperhidrosis, treatment of sweat glands, treatment of acne, and treatment of sebaceous glands.

9. The ultrasound treatment system according to any one of the preceding claims, further comprising a tissue parameter monitoring system coupled to said control system (202).

10. The ultrasound treatment system according to any one of the preceding claims, wherein said targeted delivery of ablative energy (220) is to a fixed depth of up to 5mm below a surface of skin (210).

## Patentansprüche

1. Ultraschallbehandlungssystem, gestaltet für nichtinvasives Facelifting und tiefe Gewebestraffung, wobei das Ultraschallbehandlungssystem umfasst:
ein Sondensystem (204), gestaltet für sowohl die Bildgebung als auch die Therapie, wobei das Sondensystem einen Bildgebungs- und Therapiewandler umfasst, der für die zielgerichtete Abgabe von ablativem Ultraschall in der Schicht des superfiziellen muskuloaponeurotischen Systems (SMAS) gestaltet ist, und wobei der Bildgebungs- und Therapiewandler ein Bildgebungselement und ein einzelnes Therapieelement umfasst,
wobei das Bildgebungselement dafür gestaltet ist, einen Bereich von Interesse (206) umfassend die SMAS-Schicht (216) abzubilden,
wobei das einzelne Therapieelement aus einem einzelnen Einzelelementwandler besteht, gestaltet für die Einzelfokus- und für die zielgerichtete Abgabe von ablativer Ultraschallenergie (220) in die SMAS-Schicht (216) in einer Tiefe von bis zu 15 mm unter der Hautoberfläche für die thermische Schädigung von Gewebe und zum Bewirken von Schrumpfung der SMAS-Schicht (216);
ein Steuerungssystem (202), das an das Sondensystem (204) gekoppelt ist, wobei das Steuerungssystem (202) mechanisches Scanning des Bildgebungs- und Therapiewandlers steuert und eine räumliche Steuerung und eine zeitliche Steuerung umfasst, wobei die räumliche Steuerung und die zeitliche Steuerung die zielgerichtete Abgabe von ablativer Ultraschallenergie (220) steuern, um die Schrumpfung des wenigstens einen Teils der SMAS-Schicht (216) zu bewirken;
einen Bewegungsmechanismus zum steuerbaren Erzeugen mehrerer Läsionen, wobei der Bildgebungs- und Therapiewandler innerhalb des Sondensystems (204) mechanisch in einer Richtung (226) gescannt wird, um Behandlungszonen (260) über einen ausgedehnten Bereich als Linie gebildet zu platzieren; und
ein Anzeigesystem (208), das an das Steuerungssystem (202) und an das Bildgebungselement des Sondensystems (204) gekoppelt ist, wobei das Anzeigesystem (208) zum Anzeigen von Bildern (224) des Bereichs von Interesse (206) gestaltet ist.

2. Ultraschallbehandlungssystem gemäß Anspruch 1, wobei das Bildgebungselement eine erste Dicke aufweist, wobei das einzelne Therapieelement eine zweite Dicke aufweist und wobei das Bildgebungselement dafür gestaltet ist, mit einer Bildgebungsfrequenz von zwischen 2 MHz und 75 MHz abzubilden, und das einzelne Therapieelement dafür gestaltet ist, mit einer Behandlungsfrequenz von zwischen 4 MHz und 15 MHz zu behandeln.

3. Ultraschallbehandlungssystem gemäß einem der vorstehenden Ansprüche, wobei das Steuerungssystem einen Prozessor umfasst, der dafür gestaltet ist, einen Algorithmus auszuführen, der funktionsfähig ist, Daten von dem Sondensystem umzuwandeln und ein dreidimensionales Bild des Bereichs von Interesse auf dem Anzeigesystem (208) anzuzeigen.

4. Ultraschallbehandlungssystem gemäß einem der vorstehenden Ansprüche, wobei der Bewegungsmechanismus wenigstens eines aus der Gruppe bestehend aus einem Beschleunigungsmesser, einem Encoder und einer Positions/Orientierungsvorrichtung umfasst.

5. Ultraschallbehandlungssystem gemäß einem der vorstehenden Ansprüche, wobei das Bildgebungselement und das Therapieelement in einem einzigen Doppelfunktions-Bildgebungs/Therapie-Wandlerelement integriert sind.

6. Ultraschallbehandlungssystem gemäß einem der vorstehenden Ansprüche, ferner umfassend eine Hilfsbildgebungsvorrichtung (1272), eine Behandlungsüberwachungsvorrichtung (1274) und ein sekundäres Therapiesystem (1276).

7. Ultraschallbehandlungssystem gemäß Anspruch 6, wobei die Hilfsbildgebungsvorrichtung (1272) wenigstens eines von einer photographischen Vorrichtung und einer optischen Modalität umfasst.

8. Ultraschallbehandlungssystem gemäß einem der vorstehenden Ansprüche, wobei das Sondensystem (204) für die zielgerichtete Abgabe der ablativen Ultraschallenergie (220) zum thermischen Schädigen eines Abschnitts von wenigstens einem von Haut (210), Dermis (212), Muskelfaszien (216) und Fettgewebe (214) zusätzlich zu der SMAS-Schicht (216) zur Zerstörung von Gewebe und zum Bewirken von Schrumpfung der SMAS-Schicht (216) für eines von einem Facelifting, Behandlung von lichtgealtertem Gewebe, Behandlung von übermäßiger Schweißentwicklung, Behandlung von Hyperhidrose, Behandlung von Schweißdrüsen, Behandlung von Akne und Behandlung von Talgdrüsen gestaltet ist

9. Ultraschallbehandlungssystem gemäß einem der vorstehenden Ansprüche, ferner umfassend ein Gewebeparameterüberwachungssystem, das an das Steuerungssystem (202) gekoppelt ist.

10. Ultraschallbehandlungssystem gemäß einem der vorstehenden Ansprüche, wobei die zielgerichtete Abgabe von ablativer Energie (220) in eine festgelegte Tiefe von bis zu 5 mm unter einer Hautoberfläche (210) erfolgt.

## Revendications

1. Système de traitement par ultrasons configuré pour des rhytidectomies non invasives et un raffermissement des tissus profonds, ledit système de traitement par ultrasons comprenant :
un système de sonde (204) configuré à la fois à des fins d'imagerie et de thérapie, dans lequel ledit système de sonde comprend un transducteur de thérapie et d'imagerie configuré pour une administration ciblée d'ultrason ablatif au sein de la couche de système aponévrotique musculaire superficiel (SMAS), et dans lequel le transducteur de thérapie et d'imagerie comprend un élément d'imagerie et un élément de thérapie unique ;
dans lequel l'élément d'imagerie est configuré de manière à imager une région d'intérêt (206) comprenant la couche de système SMAS (216) ;
dans lequel l'élément de thérapie unique se compose d'un transducteur à élément unique individuel, configuré pour une uniformisation et pour une administration ciblée d'énergie d'ultrason ablatif (220) dans ladite couche de système SMAS (216) à une profondeur allant jusqu'à 15 mm en dessous de la surface cutanée pour une lésion thermique tissulaire et pour occasionner le retrait de ladite couche de système SMAS (216) ;
un système de commande (202) couplé audit système de sonde (204), ledit système de commande (202) commandant le balayage mécanique du transducteur de thérapie et d'imagerie et comprenant une commande spatiale et une commande temporelle, ladite commande spatiale et ladite commande temporelle commandant ladite administration ciblée d'énergie d'ultrason ablatif (220) pour occasionner ledit retrait de ladite au moins une partie de ladite couche de système SMAS (216) ;
un mécanisme de mouvement pour créer, de façon commandée, des lésions multiples, dans lequel le transducteur de thérapie et d'imagerie au sein du système de sonde (204) est balayé mécaniquement dans une direction (226) en vue de positionner des zones de traitement (260) sur une zones étendue formée comme une ligne ; et
un système d'affichage (208) couplé audit système de commande (202) et audit élément d'imagerie dudit système de sonde (204), ledit système d'affichage (208) étant configuré de manière à afficher des images (224) de ladite région d'intérêt (206).

2. Système de traitement par ultrasons selon la revendication 1, dans lequel l'élément d'imagerie présente une première épaisseur, dans lequel l'élément de thérapie unique présente une seconde épaisseur, et dans lequel l'élément d'imagerie est configuré de manière à imager à une fréquence d'imagerie comprise entre 2 MHz et 75 MHz, et l'élément de thérapie unique est configuré de manière à traiter à une fréquence de traitement comprise entre 4 MHz et 15 MHz.

3. Système de traitement par ultrasons selon l'une quelconque des revendications précédentes, dans lequel le système de commande comprend un processeur configuré de manière à exécuter un algorithme exploitable de façon à convertir des données en provenance du système de sonde et à afficher une image tridimensionnelle de ladite région d'intérêt sur ledit système d'affichage (208).

4. Système de traitement par ultrasons selon l'une quelconque des revendications précédentes, dans lequel le mécanisme de mouvement comprend au moins un élément du groupe constitué par un accéléromètre, un codeur, et un dispositif de position/orientation.

5. Système de traitement par ultrasons selon l'une quelconque des revendications précédentes, dans lequel l'élément d'imagerie et l'élément de thérapie sont intégrés au sein d'un unique élément de transducteur de thérapie/imagerie à double fonction.

6. Système de traitement par ultrasons selon l'une quelconque des revendications précédentes, comprenant en outre un appareil d'imagerie auxiliaire (1272), un appareil de suivi de traitement (1274) et un système de thérapie secondaire (1276).

7. Système de traitement par ultrasons selon la revendication 6, dans lequel l'appareil d'imagerie auxiliaire (1272) comprend au moins l'un des éléments parmi un dispositif photographique et une modalité optique.

8. Système de traitement par ultrasons selon l'une quelconque des revendications précédentes, dans lequel ledit système de sonde (204) est configuré en vue d'une administration ciblée de ladite énergie d'ultrason ablatif (220) pour léser thermiquement une partie d'au moins l'un des éléments parmi la peau (210), le derme (212), le fascia musculaire (216), et le tissu adipeux (214), outre ladite couche de système SMAS (216) pour une destruction tissulaire et pour occasionner le retrait de ladite couche de système SMAS (216) pour l'une quelconque des interventions parmi une rhytidectomie, le traitement du tissu photo-vieilli, le traitement de la transpiration excessive, le traitement de l'hyperhidrose, le traitement des glandes sudoripares, le traitement de l'acné et le traitement des glandes sébacées.

9. Système de traitement par ultrasons selon l'une quelconque des revendications précédentes, comprenant en outre un système de suivi de paramètres tissulaires couplé audit système de commande (202).

10. Système de traitement par ultrasons selon l'une quelconque des revendications précédentes, dans lequel ladite administration ciblée d'énergie ablative (220) est mise en oeuvre à une profondeur fixe allant jusqu'à 5 mm en dessous d'une surface cutanée (210).
